# EUROPEAN PATENT APPLICATION

(11) **EP 4 800 117 A1**
(43) Date of publication of application: **02.09.2026**
(21) Application number: 24882463.3
(22) Date of filing: 24.10.2024
(51) Int. Cl.: C12P 19/30, C12N 1/21, C12N 15/31, C12N 15/54

(54) **METHOD FOR PRODUCING NMN AND METHOD FOR PRODUCING NAD**

(30) Priority: 24.10.2023 JP 2023182293
(71) Applicant: Oriental Yeast Co., Ltd., Tokyo 174-8505 (JP)
(72) Inventor: KINOSE, Keita, Nagahama-shi, Shiga 526-0804 (JP); MIZUGUCHI, Mitsuhiro, Nagahama-shi, Shiga 526-0804 (JP); KAWASAKI, Hisashi, Tokyo 142-0062 (JP); SUZUKI, Shunichi, Tokyo 174-8505 (JP)
(74) Representative: Vossius & Partner Patentanwälte Rechtsanwälte mbB
(86) International application number: PCT/JP2024/037946
(87) International publication number: WO 2025/089354

(57) **Abstract**

The present invention provides transformed microorganisms capable of synthesizing NMN or NAD at a high yield, and a method for producing NMN or NAD using the transformed microorganism. The method for producing NMN includes synthesizing NMN from Nam and PRPP using NAMPT within bacterial cells, wherein the NAMPT has a region consisting of a polypeptide having an amino acid sequence having 90 to 100% sequence similarity to an amino acid sequence represented by SEQ ID NO: 2 and has, on C-terminal of said amino acid sequence, an amino acid sequence having 0 to 100% sequence similarity to an amino acid sequence represented by SEQ ID NO: 3, or a region consisting of a polypeptide having an amino acid sequence having 90 to 100% sequence similarity to an amino acid sequence represented by SEQ ID NO: 5 and has, on C-terminal of said amino acid sequence, an amino acid sequence having 90 to 100% sequence similarity to an amino acid sequence represented by SEQ ID NO: 6.

## Description

### TECHNICAL FIELD

The present invention relates to a method for synthesizing nicotinamide mononucleotide (NMN) and a method for synthesizing nicotinamide adenine dinucleotide (NAD) using a microorganism.

This application claims priority based on Japanese Patent Application No. 2023-182293, filed on October 24, 2023, the contents of which are incorporated herein by reference.

### BACKGROUND ART

With the recent advancements in genetic modification technology, methods using microorganisms as reaction systems for synthetic reactions are increasingly being utilized for industrial mass production. While chemical synthesis often requires organic solvents, high-temperature and high-pressure reaction conditions, and heavy metal catalysts, utilizing the metabolic pathways possessed by microorganisms allows for the synthesis of useful organic compounds under mild conditions of room temperature and atmospheric pressure, and at relatively low cost. For example, NAD is a coenzyme for many oxidoreductases, and NMN is an intermediate metabolite in NAD biosynthesis, and various biological functions have been reported for both. Since both are compounds in high demand, there is a need to develop microorganisms that can produce them in large quantities.

In the synthesis of organic compounds using microorganisms, modification methods are employed to supplement metabolic pathways lacking in the microorganism for synthesis, or to enhance the metabolic efficiency (flux) of metabolic pathways inherently present in the microorganism by increasing the expression level of enzyme genes or introducing more active exogenous genes or their variants. Multiple synthesis pathways for NAD and NMN are known. For example, in mammalian cells, NMN is synthesized from nicotinamide (Nam) and phosphorylated ribose donors such as phosphoribosyl pyrophosphate (PRPP) by nicotinamide phosphoribosyltransferase (NAMPT: NMN nucleosidase; hereinafter referred to as NAMPT in the present specification). NAD is synthesized from NMN and adenosine triphosphate (ATP) by nicotinamide mononucleotide adenylyltransferase (hereinafter referred to as "NMN-AT" in the present specification). In this regard, Patent Document 1 describes that the synthesis efficiency of NMN can be increased by introducing a highly active NAMPT gene into microorganisms used for the synthesis of NMN and NAD and transforming them.

Microbial NAMPT, also known as NadV, is a protein that has significant homology to mammalian NAMPT and is encoded by the nadV gene. For example, Non-Patent Document 1 discloses that by introducing a NAMPT gene derived from Chitinophaga pinensis, which has high NMN synthesis activity, into Escherichia coli, a transformant capable of selectively and efficiently producing NMN from glucose and Nam can be obtained.

In bacteria such as Escherichia coli, Salmonella typhimurium, and Haemophilus ducreyi, the reaction to synthesize NAD from NMN is known to be catalyzed by the NadR protein, a product of the nadR gene (Non-Patent Document 2). These NadR proteins consist of a domain with NMN-to-NAD conversion function (NMN-AT function), a DNA-binding domain with transcriptional regulatory function (Non-Patent Document 3), and a domain with nicotinamide riboside kinase function (Non-Patent Document 4) that synthesizes NMN from nicotinamide riboside (hereinafter sometimes referred to as NR in the present specification). NadR proteins are thought to bind to DNA in an NAD-dependent manner and regulate the expression of genes involved in the synthesis of the nicotinamide skeleton (Non-Patent Document 3). However, the role of the DNA-binding domain in enzyme function and NAD synthesis efficiency remains unclear.

### Citation List

### Patent documents

Patent Document 1: PCT International Publication No. WO 2020/129997

### Non-Patent Documents

Non-Patent Document 1: Shoji, et al., Metabolic Engineering, 2021, vol.65, p. 167-177.
Non-Patent Document 2: Raffaelli et al., Journal of Bacteriology, 1999, vol. 181(17), p. 5509-5511.
Non-Patent Document 3: Penfound and Foster, Journal of Bacteriology, 1999, vol. 181(2), p. 648-655.
Non-Patent Document 4: Kurnasov et al., Journal of Bacteriology, 2002, vol. 184(24), p.6906-6917.
Non-Patent Document 5: Pao et al., MICROBIOLOGY AND MOLECULAR BIOLOGY REVIEWS, 1998, vol. 62(1), p. 1-34.
Non-Patent Document 6: Drew et al., Chemical Reviews, 2021, vol. 121, p. 5289-5335.

### SUMMARY OF INVENTION

### TECHNICAL PROBLEM

The objective of the present invention is to provide a transformed microorganism capable of synthesizing NMN or NAD at a high yield, and a method for producing NMN or NAD using the transformed microorganism.

### [Means for solving the problem]

As a result of intensive research to solve the above problems, the inventors have discovered that by expressing NadV derived from Sandaracinus amylolyticus (SaNadV) or NadV derived from Convivina intestini (CiNadV) in Escherichia coli, the amount of NMN accumulated in the microbial cells can be significantly increased, and furthermore, by expressing NadR lacking the DNA-binding domain, the amount of NAD accumulated in the microbial cells can be significantly increased, thereby completing the present invention.

That is, the invention includes the following aspects.
[1] A method for producing nicotinamide mononucleotide, which involves synthesizing the nicotinamide mononucleotide within bacterial cells, comprising:
   synthesizing nicotinamide mononucleotide from nicotinamide and phosphoribosyl pyrophosphate using nicotinamide phosphoribosyltransferase, wherein
   the nicotinamide phosphoribosyltransferase comprises:
      (a1) a polypeptide having an amino acid sequence represented by SEQ ID NO: 2 and having, on C-terminal of said amino acid sequence, the amino acid sequence represented by SEQ ID NO: 3,
      (a2) a polypeptide having an amino acid sequence having 90% or more sequence similarity to an amino acid sequence represented by SEQ ID NO: 2 and having, on C-terminal of said amino acid sequence, the amino acid sequence having 90% or more sequence similarity to an amino acid sequence represented by SEQ ID NO: 3, and having nicotinamide phosphoribosyltransferase activity,
      (a3) a polypeptide having an amino acid sequence represented by SEQ ID NO: 5 and having, on C-terminal of said amino acid sequence, the amino acid sequence represented by SEQ ID NO: 6, or
      (a4) a polypeptide having an amino acid sequence having 90% or more sequence similarity to an amino acid sequence represented by SEQ ID NO: 5 and having, on C-terminal of said amino acid sequence, an amino acid sequence having 90% or more sequence similarity to an amino acid sequence represented by SEQ ID NO: 6, and having nicotinamide phosphoribosyltransferase activity.
[2] The method for producing a nicotinamide mononucleotide according to [1], wherein the bacteria are cultured in a solution containing glucose and nicotinamide.
[3] A method for producing a nicotinamide adenine dinucleotide, which involves synthesizing the nicotinamide adenine dinucleotide within bacterial cells, comprising:
   synthesizing nicotinamide mononucleotide from nicotinamide and phosphoribosyl pyrophosphate using nicotinamide phosphoribosyltransferase, and
   synthesizing nicotinamide adenine dinucleotide from nicotinamide mononucleotide obtained and ATP using nicotinamide mononucleotide adenylyltransferase, wherein
   the nicotinamide phosphoribosyltransferase comprises:
      (a1) a polypeptide having an amino acid sequence represented by SEQ ID NO: 2 and having, on C-terminal of said amino acid sequence, an amino acid sequence represented by SEQ ID NO: 3,
      (a2) a polypeptide having an amino acid sequence having 90% or more sequence similarity to an amino acid sequence represented by SEQ ID NO: 2 and having, on C-terminal of said amino acid sequence, an amino acid sequence having 90% or more sequence similarity to an amino acid sequence represented by SEQ ID NO: 3, and having nicotinamide phosphoribosyltransferase activity,
      (a3) a polypeptide having an amino acid sequence represented by SEQ ID NO: 5 and having, on C-terminal of said amino acid sequence, an amino acid sequence represented by SEQ ID NO: 6, or
      (a4) a polypeptide having an amino acid sequence having 90% or more sequence similarity to an amino acid sequence represented by SEQ ID NO: 5 and having, on C-terminal of said amino acid sequence, an amino acid sequence having 90% or more sequence similarity to an amino acid sequence represented by SEQ ID NO: 6, and having nicotinamide phosphoribosyltransferase activity;
      the nicotinamide mononucleotide adenylyltransferase is encoded in a modified nadR gene introduced into the bacterium, and the modified nadR gene encodes a polypeptide that has a nicotinamide riboside kinase domain and a nicotinamide mononucleotide adenylyltransferase domain, but does not have a DNA-binding domain;
      the nicotinamide riboside kinase domain is:
         (b1) a polypeptide consisting of an amino acid sequence consisting of amino acids Nos. 230 to 410 of SEQ ID NO: 7, or
         (b2) a polypeptide having 90% or more sequence similarity to an amino acid sequence consisting of amino acids Nos. 230 to 410 of SEQ ID NO: 7 and having nicotinamide riboside kinase activity;
         the nicotinamide mononucleotide adenylyltransferase domain is:
            (b3) a polypeptide consisting of an amino acid sequence consisting of amino acids Nos. 63 to 229 of SEQ ID NO: 7, or
            (b4) a polypeptide having 90% or more sequence similarity to an amino acid sequence consisting of amino acids Nos. 63 to 229 of SEQ ID NO: 7 and having nicotinamide mononucleotide adenylyltransferase activity.
[4] The method for producing nicotinamide adenine dinucleotide according to [3], wherein the bacterium expresses an exogenous or endogenous transporter gene, and the synthesized nicotinamide adenine dinucleotide is secreted outside the bacterium.
[5] The method for producing a nicotinamide adenine dinucleotide according to [4], wherein
   the transporter is:
   (c1) a polypeptide consisting of an amino acid sequence represented by SEQ ID NO: 8,
   (c2) a polypeptide having 90% or more sequence similarity to an amino acid sequence represented by SEQ ID NO: 8 and having nicotinamide adenine dinucleotide secretion ability,
   (c3) a polypeptide consisting of an amino acid sequence represented by SEQ ID NO: 9,
   (c4) a polypeptide having 90% or more sequence similarity to an amino acid sequence represented by SEQ ID NO: 9 and having nicotinamide adenine dinucleotide secretion ability,
   (c5) a polypeptide consisting of an amino acid sequence represented by SEQ ID NO: 10,
   (c6) a polypeptide having 90% or more sequence similarity to an amino acid sequence represented by SEQ ID NO: 10 and having nicotinamide adenine dinucleotide secretion ability,
   (c7) a polypeptide consisting of an amino acid sequence represented by SEQ ID NO: 11,
   (c8) a polypeptide having 90% or more sequence similarity to an amino acid sequence represented by SEQ ID NO: 11 and having nicotinamide adenine dinucleotide secretion ability,
   (c9) a polypeptide consisting of an amino acid sequence represented by SEQ ID NO: 12,
   (c10) a polypeptide having 90% or more sequence similarity to an amino acid sequence represented by SEQ ID NO: 12 and having nicotinamide adenine dinucleotide secretion ability,
   (c11) a polypeptide consisting of an amino acid sequence represented by SEQ ID NO: 13,
   (c12) a polypeptide having 90% or more sequence similarity to an amino acid sequence represented by SEQ ID NO: 13 and having nicotinamide adenine dinucleotide secretion ability,
   (c13) a polypeptide consisting of an amino acid sequence represented by SEQ ID NO: 14,
   (c14) a polypeptide having 90% or more sequence similarity to an amino acid sequence represented by SEQ ID NO: 14 and having nicotinamide adenine dinucleotide secretion ability,
   (c15) a polypeptide consisting of an amino acid sequence represented by SEQ ID NO: 15,
   (c16) a polypeptide having 90% or more sequence similarity to an amino acid sequence represented by SEQ ID NO: 15 and having nicotinamide adenine dinucleotide secretion ability,
   (c17) a polypeptide consisting of an amino acid sequence represented by SEQ ID NO: 16,
   (c18) a polypeptide having 90% or more sequence similarity to an amino acid sequence represented by SEQ ID NO: 16 and having nicotinamide adenine dinucleotide secretion ability,
   (c19) a polypeptide consisting of an amino acid sequence represented by SEQ ID NO: 17,
   (c20) a polypeptide having 90% or more sequence similarity to an amino acid sequence represented by SEQ ID NO: 17 and having nicotinamide adenine dinucleotide secretion ability,
   (c21) a polypeptide consisting of an amino acid sequence represented by SEQ ID NO: 18,
   (c22) a polypeptide having 90% or more sequence similarity to an amino acid sequence represented by SEQ ID NO: 18 and having nicotinamide adenine dinucleotide secretion ability,
   (c23) a polypeptide consisting of an amino acid sequence represented by SEQ ID NO: 19,
   (c24) a polypeptide having 90% or more sequence similarity to an amino acid sequence represented by SEQ ID NO: 19 and having nicotinamide adenine dinucleotide secretion ability,
   (c25) a polypeptide consisting of an amino acid sequence represented by SEQ ID NO: 20,
   (c26) a polypeptide having 90% or more sequence similarity to an amino acid sequence represented by SEQ ID NO: 20 and having nicotinamide adenine dinucleotide secretion ability,
   (c27) a polypeptide consisting of an amino acid sequence represented by SEQ ID NO: 21,
   (c28) a polypeptide having 90% or more sequence similarity to an amino acid sequence represented by SEQ ID NO: 21 and having nicotinamide adenine dinucleotide secretion ability,
   (c29) a polypeptide consisting of an amino acid sequence represented by SEQ ID NO: 22,
   (c30) a polypeptide having 90% or more sequence similarity to an amino acid sequence represented by SEQ ID NO: 22 and having nicotinamide adenine dinucleotide secretion ability,
   (c31) a polypeptide consisting of an amino acid sequence represented by SEQ ID NO: 23,
   (c32) a polypeptide having 90% or more sequence similarity to an amino acid sequence represented by SEQ ID NO: 23 and having nicotinamide adenine dinucleotide secretion ability,
   (c33) a polypeptide consisting of an amino acid sequence represented by SEQ ID NO: 24,
   (c34) a polypeptide having 90% or more sequence similarity to an amino acid sequence represented by SEQ ID NO: 24 and having nicotinamide adenine dinucleotide secretion ability,
   (c35) a polypeptide consisting of an amino acid sequence represented by SEQ ID NO: 25,
   (c36) a polypeptide having 90% or more sequence similarity to an amino acid sequence represented by SEQ ID NO: 25 and having nicotinamide adenine dinucleotide secretion ability,
   (c37) a polypeptide consisting of an amino acid sequence represented by SEQ ID NO: 26,
   (c38) a polypeptide having 90% or more sequence similarity to an amino acid sequence represented by SEQ ID NO: 26 and having nicotinamide adenine dinucleotide secretion ability,
   (c39) a polypeptide consisting of an amino acid sequence represented by SEQ ID NO: 27,
   (c40) a polypeptide having 90% or more sequence similarity to an amino acid sequence represented by SEQ ID NO: 27 and having nicotinamide adenine dinucleotide secretion ability,
   (c41) a polypeptide consisting of an amino acid sequence represented by SEQ ID NO: 28, or
   (c42) a polypeptide having 90% or more sequence similarity to an amino acid sequence represented by SEQ ID NO: 28 and having nicotinamide adenine dinucleotide secretion ability.
[6] The method for producing a nicotinamide adenine dinucleotide according to [4], wherein the transporter is MFS transporter having an SLDQ motif or an SLEQ motif in the first transmembrane domain.
[7] A transformed microorganism, which is a microbial transformant, into which an exogenous nicotinamide phosphoribosyltransferase gene has been introduced, wherein
   the nicotinamide phosphoribosyltransferase comprises:
   (a1) a polypeptide having an amino acid sequence represented by SEQ ID NO: 2 and having, on C-terminal of said amino acid sequence, an amino acid sequence represented by SEQ ID NO: 3,
   (a2) a polypeptide having an amino acid sequence having 90% or more sequence similarity to an amino acid sequence represented by SEQ ID NO: 2 and having, on C-terminal of said amino acid sequence, an amino acid sequence having 90% or more sequence similarity to an amino acid sequence represented by SEQ ID NO: 3, and having nicotinamide phosphoribosyltransferase activity,
   (a3) a polypeptide having an amino acid sequence represented by SEQ ID NO: 5 and having, on C-terminal of said amino acid sequence, an amino acid sequence represented by SEQ ID NO: 6, or
   (a4) a polypeptide having an amino acid sequence having 90% or more sequence similarity to an amino acid sequence represented by SEQ ID NO: 5 and having, on C-terminal of said amino acid sequence, an amino acid sequence having 90% or more sequence similarity to an amino acid sequence represented by SEQ ID NO: 6, and having nicotinamide phosphoribosyltransferase activity.
[8] The transformed microorganism according to [7], wherein
   a modified nadR gene has been further introduced;
   the modified nadR gene encodes a polypeptide that has a nicotinamide riboside kinase domain and a nicotinamide mononucleotide adenylyltransferase domain, but does not have a DNA-binding domain;
   the nicotinamide riboside kinase domain is:
      (b1) a polypeptide consisting of an amino acid sequence consisting of amino acids 230 to 410 of SEQ ID NO: 7, or
      (b2) a polypeptide having 90% or more sequence similarity to an amino acid sequence consisting of amino acids 230 to 410 of SEQ ID NO: 7 and having nicotinamide riboside kinase activity;
      the nicotinamide mononucleotide adenylyltransferase domain is:
         (b3) a polypeptide consisting of an amino acid sequence consisting of amino acids 63 to 229 of SEQ ID NO: 7, or
         (b4) a polypeptide having 90% or more sequence similarity to an amino acid sequence consisting of amino acids 63 to 229 of SEQ ID NO: 7 and having nicotinamide mononucleotide adenylyltransferase activity.
[9] The transformed microorganism according to [7] or [8], wherein
   an exogenous or endogenous transporter gene has been further introduced; and
   the transporter is:
      (c1) a polypeptide consisting of an amino acid sequence represented by SEQ ID NO: 8,
      (c2) a polypeptide having 90% or more sequence similarity to an amino acid sequence represented by SEQ ID NO: 8 and having nicotinamide adenine dinucleotide secretion ability,
      (c3) a polypeptide consisting of an amino acid sequence represented by SEQ ID NO: 9,
      (c4) a polypeptide having 90% or more sequence similarity to an amino acid sequence represented by SEQ ID NO: 9 and having nicotinamide adenine dinucleotide secretion ability,
      (c5) a polypeptide consisting of an amino acid sequence represented by SEQ ID NO: 10,
      (c6) a polypeptide having 90% or more sequence similarity to an amino acid sequence represented by SEQ ID NO: 10 and having nicotinamide adenine dinucleotide secretion ability,
      (c7) a polypeptide consisting of an amino acid sequence represented by SEQ ID NO: 11,
      (c8) a polypeptide having 90% or more sequence similarity to an amino acid sequence represented by SEQ ID NO: 11 and having nicotinamide adenine dinucleotide secretion ability,
      (c9) a polypeptide consisting of an amino acid sequence represented by SEQ ID NO: 12,
      (c10) a polypeptide having 90% or more sequence similarity to an amino acid sequence represented by SEQ ID NO: 12 and having nicotinamide adenine dinucleotide secretion ability,
      (c11) a polypeptide consisting of an amino acid sequence represented by SEQ ID NO: 13,
      (c12) a polypeptide having 90% or more sequence similarity to an amino acid sequence represented by SEQ ID NO: 13 and having nicotinamide adenine dinucleotide secretion ability,
      (c13) a polypeptide consisting of an amino acid sequence represented by SEQ ID NO: 14,
      (c14) a polypeptide having 90% or more sequence similarity to an amino acid sequence represented by SEQ ID NO: 14 and having nicotinamide adenine dinucleotide secretion ability,
      (c15) a polypeptide consisting of an amino acid sequence represented by SEQ ID NO: 15,
      (c16) a polypeptide having 90% or more sequence similarity to an amino acid sequence represented by SEQ ID NO: 15 and having nicotinamide adenine dinucleotide secretion ability,
      (c17) a polypeptide consisting of an amino acid sequence represented by SEQ ID NO: 16,
      (c18) a polypeptide having 90% or more sequence similarity to an amino acid sequence represented by SEQ ID NO: 16 and having nicotinamide adenine dinucleotide secretion ability,
      (c19) a polypeptide consisting of an amino acid sequence represented by SEQ ID NO: 17,
      (c20) a polypeptide having 90% or more sequence similarity to an amino acid sequence represented by SEQ ID NO: 17 and having nicotinamide adenine dinucleotide secretion ability,
      (c21) a polypeptide consisting of an amino acid sequence represented by SEQ ID NO: 18,
      (c22) a polypeptide having 90% or more sequence similarity to an amino acid sequence represented by SEQ ID NO: 18 and having nicotinamide adenine dinucleotide secretion ability,
      (c23) a polypeptide consisting of an amino acid sequence represented by SEQ ID NO: 19,
      (c24) a polypeptide having 90% or more sequence similarity to an amino acid sequence represented by SEQ ID NO: 19 and having nicotinamide adenine dinucleotide secretion ability,
      (c25) a polypeptide consisting of an amino acid sequence represented by SEQ ID NO: 20,
      (c26) a polypeptide having 90% or more sequence similarity to an amino acid sequence represented by SEQ ID NO: 20 and having nicotinamide adenine dinucleotide secretion ability,
      (c27) a polypeptide consisting of an amino acid sequence represented by SEQ ID NO: 21,
      (c28) a polypeptide having 90% or more sequence similarity to an amino acid sequence represented by SEQ ID NO: 21 and having nicotinamide adenine dinucleotide secretion ability,
      (c29) a polypeptide consisting of an amino acid sequence represented by SEQ ID NO: 22,
      (c30) a polypeptide having 90% or more sequence similarity to an amino acid sequence represented by SEQ ID NO: 22 and having nicotinamide adenine dinucleotide secretion ability,
      (c31) a polypeptide consisting of an amino acid sequence represented by SEQ ID NO: 23,
      (c32) a polypeptide having 90% or more sequence similarity to an amino acid sequence represented by SEQ ID NO: 23 and having nicotinamide adenine dinucleotide secretion ability,
      (c33) a polypeptide consisting of an amino acid sequence represented by SEQ ID NO: 24,
      (c34) a polypeptide having 90% or more sequence similarity to an amino acid sequence represented by SEQ ID NO: 24 and having nicotinamide adenine dinucleotide secretion ability,
      (c35) a polypeptide consisting of an amino acid sequence represented by SEQ ID NO: 25,
      (c36) a polypeptide having 90% or more sequence similarity to an amino acid sequence represented by SEQ ID NO: 25 and having nicotinamide adenine dinucleotide secretion ability,
      (c37) a polypeptide consisting of an amino acid sequence represented by SEQ ID NO: 26,
      (c38) a polypeptide having 90% or more sequence similarity to an amino acid sequence represented by SEQ ID NO: 26 and having nicotinamide adenine dinucleotide secretion ability,
      (c39) a polypeptide consisting of an amino acid sequence represented by SEQ ID NO: 27,
      (c40) a polypeptide having 90% or more sequence similarity to an amino acid sequence represented by SEQ ID NO: 27 and having nicotinamide adenine dinucleotide secretion ability,
      (c41) a polypeptide consisting of an amino acid sequence represented by SEQ ID NO: 28, or
      (c42) a polypeptide having 90% or more sequence similarity to an amino acid sequence represented by SEQ ID NO: 28 and having nicotinamide adenine dinucleotide secretion ability.
[10] The transformed microorganism according to [7] or [8], wherein
   an exogenous or endogenous transporter gene has been further introduced, and
   the transporter is an MFS transporter having an SLDQ motif or an SLEQ motif in the first transmembrane domain.

### Advantageous Effects of Invention

The present invention provides a transformant capable of producing NMN and NAD in large quantities. Furthermore, NMN and NAD can be produced at a high yield simply by culturing the transformant.

### DESCRIPTION OF EMBODIMENTS

In the present invention and the present specification, the term "NAD" comprehensively refers to both the oxidized form (NAD⁺) and the reduced form (NADH), unless otherwise specified.

In the invention and the specification, the term "transformed microorganism" means a microorganism into which a desired gene has been introduced in such a way that the structural protein encoded by that gene can be expressed. Furthermore, unless otherwise specified, the term "host" refers to a microorganism into which a desired exogenous gene has been introduced.

### <Method for producing NMN and transformed microorganisms used therein>

The method for producing NMN in the embodiment is a method for synthesizing NMN within bacterial cells, wherein NMN is synthesized from Nam and PRPP using NAMPT, and an enzyme consisting of the NAMPT catalytic region of Sa_NadV or an enzyme consisting of the NAMPT catalytic region of Ci_NadV as NAMPT.

NadV is a protein that has two conserved domains, one at the N-terminal and one at the C-terminal. Having both N-terminal and C-terminal domains gives NadV NAMPT activity.

Sa_NadV is a protein consisting of 463 amino acids in total length. The amino acid sequence of this full-length protein is registered in the protein database UniProt (https://www.uniprot.org) (A0A0F6W0P6). The polypeptide region from amino acids Nos. 8 to 105 of Sa_NadV is the N-terminal domain, and the polypeptide region from amino acids 176 to 419 is the C-terminal domain.

Ci_NadV is a protein consisting of 462 amino acids in full length, and its full-length amino acid sequence is registered in the UniProt database (A0A2U1D921). The polypeptide region from amino acids Nos. 3 to 98 of Ci_NadV is the N-terminal domain, and the polypeptide region from amino acids Nos. 171 to 442 is the C-terminal domain.

**[Table 1]**

| | Amino acid sequence | SEQ ID NO |
|---|---|---|
| Full-length protein of Sa_NadV | MITMEGSNLLLGTDSYKVTHWRQYPPGTTRVSSFLESRGGV YPSVVFFGLQYLLDRHFAGVVVTEDRIAEAREIFAAHFGDPT LFHEAGWRHVLDAHGGRLPVEIRAVPEGTVVPVSNVLMTIE NTDPRVPWLTNYLESLLLQVWYPCTVATQSREMRRTITAHL EKTGTPSLVGFELHDFGLRGSTSHESAAIGGAAHLVSFEGTD TIPALLLARRHYGEPMAGFSIPAAEHSTITSWGRDRESSAYRN MLESYPRGLVAVVSDSYDVYRACEQLWGRELRELVLSRDGT LVVRPDSGHPPEVVCRVLEILGRAFGTAINAKGYRVLDPHVR VIQGDGIDPVMIAEILAAMSERGWSADNVAFGSGGALLQKV HRDTQNLAIKCSAVEIDGAWRPVMKDPVTDPGKRSKSGRLA LVREAGAFRTVARGDSGHPGDLLEPVFRDGEILRRQTLSDIR RRALEG* | 1 |
| N-terminal domain of Sa_NadV | NLLLGTDSYKVTHWRQYPPGTTRVSSFLESRGGVYPSVVFFG LQYLLDRHFAGVVVTEDRIAEAREIFAAHFGDPTLFHEAGWR HVLDAHGGRLPVEI | 2 |
| C-terminal domain of Sa_NadV | FELHDFGLRGSTSHESAAIGGAAHLVSFEGTDTIPALLLARRH YGEPMAGFSIPAAEHSTITSWGRDRESSAYRNMLESYPRGLV AVVSDSYDVYRACEQLWGRELRELVLSRDGTLVVRPDSGHP PEVVCRVLEILGRAFGTAINAKGYRVLDPHVRVIQGDGIDPV MIAEILAAMSERGWSADNVAFGSGGALLQKVHRDTQNLAIK CSAVEIDGAWRPVMKDPVTDPGKRSKSGRLALVRE | 3 |
| Full-length protein of Ci_NadV | MTNIILATDAYKLTHHLQYPQNITKLYSYGEARQGGRYDHIS WFGMSMILNDYFTQPVTDAMIDEAQKVAELTFGNDVYFNRP VWEKVRDLGHFPVKIKALPEGMVVPTGTALFTIESTQSWFAT SLNALETILMHVWYPTTIATNSMYIKKRLAPLVEKSGSMDNL PLMVNDFGLRGATSLESGERGGSAHLLHFRGSDNMAASRYF ASVYGLEGRALSIWATEHSVATAYGEGQGELDYLNAQLDRT PEEAPVSIVIDSYDTFNFVQNVVGSPAMKERIKNRLGRVVLR | 4 |
| | PDSGDPQTVDLQILDLLAEIFGTSLNEKGYRVLNHNVGLIQG DGMNADTMIELYQALLDHGWSSDNLVVGSGGGLLQEGFTR DTERFAIKEAYAENSAGEHLQIQKRPKTDPSKVSKSGRMKVI YDDQGQIITVPADHPGQDLLETVYDNGYMKQIDVNSMLARA ENFHDF* | |
| N-terminal domain of Ci_NadV | NIILATDAYKLTHHLQYPQNITKLYSYGEARQGGRYDHISWF GMSMILNDYFTQPVTDAMIDEAQKVAELTFGNDVYFNRPV WEKVRDLGHFPVKI | 5 |
| C-terminal domain of Ci_NadV | VNDFGLRGATSLESGERGGSAHLLHFRGSDNMAASRYFASV YGLEGRALSIWATEHSVATAYGEGQGELDYLNAQLDRTPEE APVSIVIDSYDTFNFVQNVVGSPAMKERIKNRLGRVVLRPDS GDPQTVDLQILDLLAEIFGTSLNEKGYRVLNHNVGLIQGDGM NADTMIELYQALLDHGWSSDNLVVGSGGGLLQEGFTRDTER FAIKEAYAENSAGEHLQIQKRPKTDPSKVSKSGRMKVIYDDQ GQIITVPADHPGQDLLETVYDNG | 6 |

The NAMPT used in the method for producing NMN of the embodiment is specifically a protein having a region consisting of any of the polypeptides (a1) to (a4) below.
(a1) a polypeptide having an amino acid sequence represented by SEQ ID NO: 2 and having, on C-terminal of said amino acid sequence, the amino acid sequence represented by SEQ ID NO: 3.
(a2) a polypeptide having an amino acid sequence having 90% or more sequence similarity to an amino acid sequence represented by SEQ ID NO: 2 and having, on C-terminal of said amino acid sequence, the amino acid sequence having 90% or more sequence similarity to an amino acid sequence represented by SEQ ID NO: 3, and having nicotinamide phosphoribosyltransferase activity.
(a3) a polypeptide having an amino acid sequence represented by SEQ ID NO: 5 and having, on C-terminal of said amino acid sequence, the amino acid sequence represented by SEQ ID NO: 6.
(a4) a polypeptide having an amino acid sequence having 90% or more sequence similarity to an amino acid sequence represented by SEQ ID NO: 5 and having, on C-terminal of said amino acid sequence, an amino acid sequence having 90% or more sequence similarity to an amino acid sequence represented by SEQ ID NO: 6, and having nicotinamide phosphoribosyltransferase activity.

In the invention and the specification, the term "polypeptide" refers to a polymer of 20 or more amino acids bonded together by peptide bonds, and the term "peptide" refers to a polymer of 2 to 19 amino acids bonded together by peptide bonds.

Generally, proteins (polypeptides) that have some kind of biological activity can have one or more amino acids deleted, substituted, or added without impairing their biological activity. In other words, both Sa_NadV and Ci_NadV can have one or more amino acids deleted, substituted, or added without losing their NAMPT activity.

In the invention and the specification, the phrase "deletion of amino acids in a polypeptide" means that some of the amino acid residues constituting the polypeptide are lost (removed).

In the invention and the specification, the phrase "substitution of amino acids in a polypeptide" means that some of the amino acid residues constituting the polypeptide are replaced with other amino acid residues.

In the invention and the specification, the phrase "addition of amino acids to a polypeptide" means that some new amino acid residues are inserted into the polypeptide.

Sequence similarity between two amino acid sequences can be determined through performing a global alignment of the sequences using the Needleman-Wunsch algorithm, well-known technique. Here, the amino acid substitution score can be defined using the common matrix BLOSUM62. The similarity between the two amino acid sequences is calculated as a percentage of the proportion of similar amino acids in the obtained alignment. Here, similar amino acids are defined as those with positive scores in the substitution matrix.

The polypeptide in (a1) is not particularly limited, as long as it has the N-terminal domain of Sa_NadV (SEQ ID NO: 2) (hereinafter sometimes referred to as "Sa_NadV_N-terminal domain") and the C-terminal domain of Sa_NadV (SEQ ID NO: 3) (hereinafter sometimes referred to as "Sa_NadV_C-terminal domain"). The polypeptide in (a1) may be a polypeptide in which the Sa_NadV_N-terminal domain and the Sa_NadV_C-terminal domain are directly linked, or the two domains may be linked by a linker consisting of peptides or polypeptides comprising one or more amino acids. In addition, the polypeptide in (a1) may have a peptide or polypeptide linked to N-terminal of the Sa_NadV_N-terminal domain or C-terminal of the Sa_NadV_C-terminal domain. An example of the polypeptide in (a1) is the full-length protein of Sa_NadV.

In the amino acid sequence of (a2) above, the sequence similarity to the amino acid sequence represented by Sequence ID No. 2 is not particularly limited as long as it is 90% or more and less than 100%, and it is preferably 95% or more and less than 100%, and more preferably 98% or more and less than 100%. Furthermore, in the amino acid sequence of (a2) above, the sequence similarity to the amino acid sequence represented by Sequence ID No. 2 may be 70% or more, or 80% or more.

In the amino acid sequence of (a2) above, the sequence similarity to the amino acid sequence represented by Sequence ID No. 3 is not particularly limited as long as it is 90% or more and less than 100%, and it is preferably 95% or more and less than 100%, and more preferably 98% or more and less than 100%. Furthermore, in the amino acid sequence of (a2) above, the sequence similarity to the amino acid sequence represented by Sequence ID No. 3 may be 70% or more, or 80% or more.

The polypeptide in (a2) may be a polypeptide having an amino acid sequence having 90% or more but less than 100%, preferably 95% or more but less than 100%, and more preferably 98% or more but less than 100% sequence identity to the amino acid sequence represented by SEQ ID NO: 2, and further having, on C-ternila of said amino acid sequence, an amino acid sequence having 90% or more but less than 100%, preferably 95% or more but less than 100%, and more preferably 98% or more but less than 100% sequence identity to the amino acid sequence represented by SEQ ID NO: 3, and having NAMPT activity.

Sequence identity between two amino acid sequences is determined through aligning the two amino acid sequences such that the number of identical amino acid residues is maximized, while introducing gaps in portions corresponding to insertions and deletions, and calculating the percentage of matching amino acid residues relative to the total amino acid sequence excluding gaps in the resulting alignment. The sequence identity between amino acid sequences can be determined using various homology search software known in the art. For example, the value of sequence identity between amino acid sequences can be calculated based on the alignment obtained using the known homology search software BLASTP.

The polypeptide (a2) is not particularly limited as long as it has an N-terminal domain (hereinafter sometimes referred to as the "mutant Sa_NadV_N-terminal domain") consisting of an amino acid sequence with 90% or more sequence similarity to the Sa_NadV_N-terminal domain (SEQ ID NO: 2) and a C-terminal domain (hereinafter sometimes referred to as the "mutant Sa_NadV_C-terminal domain") consisting of an amino acid sequence with 90% or more sequence similarity to the Sa_NadV_C-terminal domain (SEQ ID NO: 3), and has NAMPT activity. The polypeptide (a2) may be a polypeptide in which the mutant Sa_NadV_N-terminal domain and the mutant Sa_NadV_C-terminal domain are directly linked, or both domains may be linked via a linker consisting of a peptide or polypeptide of one or more amino acids. Furthermore, the polypeptide (a2) may have a peptide or polypeptide linked to the N-terminus of the mutant Sa_NadV_N-terminal domain or the C-terminus of the mutant Sa_NadV_C-terminal domain. Examples of the polypeptide (a2) include a polypeptide consisting of an amino acid sequence having 90% or more sequence similarity to an amino acid sequence of the full-length protein of Sa_NadV (SEQ ID NO: 1) and having NAMPT activity.

The polypeptide (a3) is not particularly limited as long as it has the N-terminal domain of Ci_NadV (SEQ ID NO: 5) (hereinafter sometimes referred to as the "Ci_NadV_N-terminal domain") and the C-terminal domain of Ci_NadV (SEQ ID NO: 6) (hereinafter sometimes referred to as the "Ci_NadV_C-terminal domain"). The polypeptide (a3) may be a polypeptide in which the Ci_NadV_N-terminal domain and the Ci_NadV_C-terminal domain are directly linked, or both domains may be linked via a linker consisting of a peptide or polypeptide of one or more amino acids. Furthermore, the polypeptide (a3) may have a peptide or polypeptide linked to the N-terminus of the Ci_NadV_N-terminal domain or the C-terminus of the Ci_NadV_C-terminal domain. An example of the polypeptide (a3) is the full-length Ci_NadV protein.

The sequence similarity of the amino acid sequence of (a4) to the amino acid sequence represented by SEQ ID NO: 5 is not particularly limited as long as it is 90% or more but less than 100%, and is preferably 95% or more but less than 100%, and more preferably 98% or more but less than 100%. Furthermore, the sequence similarity of the amino acid sequence of (a4) to the amino acid sequence represented by SEQ ID NO: 5 may be 70% or more, or may be 80% or more.

The sequence similarity of the amino acid sequence of (a4) to the amino acid sequence represented by SEQ ID NO: 6 is not particularly limited as long as it is 90% or more but less than 100%, but is preferably 95% or more but less than 100%, and more preferably 98% or more but less than 100%. Furthermore, the sequence similarity of the amino acid sequence of (a4) to the amino acid sequence represented by SEQ ID NO: 6 may be 70% or more, or may be 80% or more.

The polypeptide (a4) may be a polypeptide having an amino acid sequence having 90% to less than 100%, preferably 95% to less than 100%, and more preferably 98% to less than 100% sequence identity to the amino acid sequence shown in SEQ ID NO: 5 and further having, on C-terminal of said amino acid sequence, an amino acid sequence having 90% to less than 100%, preferably 95% to less than 100%, and more preferably 98% to less than 100% sequence identity to the amino acid sequence represented by SEQ ID NO: 6, and having NAMPT activity.

The polypeptide (a4) is not particularly limited as long as it has an N-terminal domain (hereinafter sometimes referred to as the "mutant Ci_NadV_N-terminal domain") consisting of an amino acid sequence with 90% or more sequence similarity to the Ci_NadV_N-terminal domain (SEQ ID NO: 5) and a C-terminal domain (hereinafter sometimes referred to as the "mutant Ci_NadV_C-terminal domain") consisting of an amino acid sequence with 90% or more sequence similarity to the Ci_NadV_C-terminal domain (SEQ ID NO: 6), and has NAMPT activity. The polypeptide (a4) may be a polypeptide in which the mutant Ci_NadV_N-terminal domain and the mutant Ci_NadV_C-terminal domain are directly linked, or both domains may be linked via a linker consisting of a peptide or polypeptide of one or more amino acids. Furthermore, the polypeptide (a4) may have a peptide or polypeptide linked to the N-terminus of the mutant Ci_NadV_N-terminal domain or the C-terminus of the mutant Ci_NadV_C-terminal domain. Examples of the polypeptide (a4) include a polypeptide consisting of an amino acid sequence having 90% or more sequence similarity to an amino acid sequence of the full-length protein of Ci_NadV (SEQ ID NO: 1) and having NAMPT activity.

The polypeptides (a1) to (a4) may have various functional domains not found in Sa_NadV or Ci_NadV, as long as they retain NAMPT activity in host cells. Examples of such functional domains include various signal peptides and various tag peptides. These signal peptides and tag peptides can be appropriately selected from various signal peptides and tag peptides commonly used in the production of recombinant proteins. Examples of such tag peptides include tags commonly used in the expression or purification of recombinant proteins, such as His tags, HA (hemagglutinin) tags, Myc tags, and Flag tags.

In the method for producing NMN in the present embodiment, NMN is synthesized by a transformed microorganism (hereinafter sometimes referred to as "NAMPT-transformed microorganism") into which a gene encoding a protein having a NAMPT catalytic region consisting of one of the polypeptides (a1) to (a4) has been introduced into the host bacteria. The polynucleotide encoding the protein having a NAMPT catalytic region consisting of polypeptides (a1) to (a4) is not particularly limited, but it is preferable to select a degenerate codon that is frequently used in the host. Modifying the base sequence encoding the target polypeptide to a codon that is frequently used in the host without changing the amino acid sequence of the target polypeptide can be done by known gene sequence mutation techniques or artificial gene synthesis methods.

NAMPT-introduced microorganisms are obtained by introducing an expression vector into a host bacterium that incorporates a gene encoding a protein having a NAMPT catalytic region consisting of one of the polypeptides (a1) to (a4), in a state that allows NAMPT to be expressed in the host cell. Such expression vectors can be produced by known gene sequence mutation techniques or artificial gene synthesis methods. For example, such an expression vector can be produced by incorporating an expression cassette consisting of, from upstream, DNA having a promoter sequence, a polynucleotide encoding a protein having NAMPT catalytic region consisting of one of the polypeptides (a1) to (a4), and DNA having a terminator sequence into a plasmid commonly used as an expression vector. Specific examples of such promoters include the T7 promoter, lambda PR promoter, lac promoter, tac promoter, and pBAD/AraC promoter. The incorporation of a polynucleotide encoding a protein having a NAMPT catalytic region consisting of one of the polypeptides (a1) to (a4) and an expression cassette containing it into an expression vector can be carried out using well-known genetic engineering techniques, or a commercially available expression vector production kit may be used.

The expression vector for expressing a protein having a NAMPT catalytic domain composed of any polypeptide (a1) to (a4) is preferably an expression vector that incorporates not only an expression cassette for a protein having a NAMPT catalytic domain composed of any polypeptide (a1) to (a4), but also a drug resistance gene. Examples of such drug resistance genes include ampicillin resistance genes, kanamycin resistance genes, hygromycin resistance genes, and chloramphenicol resistance genes.

The expression vector incorporating the expression cassette of a protein having a NAMPT catalytic region consisting of any polypeptide (a1) to (a4) is not particularly limited, and commonly used vectors such as plasmids and phages can be used. Specifically, the examples include pET, pSTV29, pMW218, pUC18, and pBR322.

The host bacteria are not particularly limited, and those skilled in the art can appropriately select and use bacteria that can be expected to produce similar effects. These bacteria may be Gram-negative or Gram-positive. Examples of Gram-negative bacteria include Escherichia species such as Escherichia coli. Examples of Gram-positive bacteria include Bacillus species such as Bacillus subtilis, and Corynebacterium species such as Corynebacterium glutamicum, Corynebacterium stationis, Corynebacterium tuberculostearicum, Corynebacterium sp. p3-SID1194, Corynebacterium genitalium, Corynebacterium pseudotuberculosis, and Corynebacterium kroppenstedtii. In the method for producing NMN according to the present embodiment, it is preferable to appropriately select and use bacteria from among the Escherichia species commonly used in culture, with Escherichia coli being more preferable, and Escherichia coli K12 and its derived strains or Escherichia coli B and its derived strains being even more preferable.

The host bacteria may be natural bacteria, that is, bacteria that have not been artificially genetically modified, and may have undergone genetic modification other than the introduction of an expression cassette of a protein having a NAMPT catalytic region consisting of any polypeptide (a1) to (a4).

The method for producing transformed microorganisms using an expression vector for expressing a protein having a NAMPT catalytic region consisting of any polypeptide (a1) to (a4) is not particularly limited and can be carried out by methods commonly used when producing transformants. Examples of such methods include electroporation, calcium chloride method, and PEG (polyethylene glycol) method.

The NAMPT-introduced microorganisms can be cultured in the same manner as the host bacteria. NAMPT-introduced microorganisms can be cultured at 18-37°C in a culture medium containing nutrients necessary for growth, such as carbon source, nitrogen source, and minerals. As the culture media, commonly used media for culturing Escherichia coli, such as LB (Lysogeny Broth) medium, minimal salt (M9) medium, TB (Terrific Broth) medium, SOB medium, SOC medium, 2×YT medium, and NZCYM liquid medium, can be used, modified as needed.

In the method for producing NMN in the present embodiment, NMN is synthesized intracellularly within the NAMPT-introduced microorganism using the introduced NAMPT (a protein having NAMPT catalytic region consisting of any of the polypeptides (a1) to (a4)) from Nam and PRPP. Nam and PRPP may be incorporated directly into the NAMPT-introduced microorganism, or they may be synthesized within the cell of the NAMPT-introduced microorganism. For example, PRPP is synthesized from glucose taken in externally by the transformed microorganism using the metabolic pathway of the transformed microorganism.

For example, NMN can be produced by culturing the NAMPT-introduced microorganism in a solution containing Nam and glucose under conditions that allow the introduced NAMPT to be expressed. The solution may be a culture medium, and a buffer containing glucose and Nam is preferable because it facilitates the purification of the produced NMN.

In transformed microorganisms into which an expression cassette incorporating a promoter that does not have the ability to control the timing of expression, etc., NAMPT (a protein having NAMPT catalytic region consisting of any polypeptide (a1) to (a4)) encoded by a polynucleotide incorporated downstream of the promoter is constitutively expressed. On the other hand, in transformed microorganisms into which a so-called expression-inducing promoter is incorporated, which induces expression by specific compounds or temperature conditions, etc., an induction treatment suitable for each expression induction condition is performed to induce the expression of a protein having a NAMPT catalytic region consisting of any polypeptide (a1) to (a4) in the transformed organism.

### <Method for Producing NAD and Transformed Microorganism Used Therefor>

The method for producing NAD in the embodiment is a method for synthesizing NAD within bacterial cells wherein NMN is synthesized from Nam and PRPP by NAMPT, and NAD is synthesized from the obtained NMN and ATP by NMN-AT, wherein the NAMPT is a protein having NAMPT catalytic region consisting of any polypeptide from (a1) to (a4) above, and furthermore, NMN-AT encoded by a modified nadR gene that lacks a DNA-binding domain is used. This modified nadR gene has a nicotinamide riboside kinase domain and a nicotinamide mononucleotide adenylyltransferase domain, but encodes a polypeptide that has been modified to delete a DNA-binding domain. In the present specification, the polypeptide encoded by this modified nadR gene may be referred to as a DNA-binding domain-deficient mutant of NadR.

The NMN-AT used in the NAD production method of the embodiment is a DNA-binding domain-deficient mutant of NadR. Compared to expressing the full-length NadR protein or only the NMN-AT domain, NAD productivity is dramatically increased. When a mutant in which only the nicotinamide riboside kinase domain is deleted from the full-length NadR protein is expressed, such an effect of improving NAD productivity cannot be obtained.

Escherichia coli NadR (Ec_NadR) is a protein consisting of 410 amino acids, and its full-length amino acid sequence has been registered in the UniProt database (P272786). The region from amino acids 7 to 62 of Ec_NadR is the DNA-binding (HTH cro/C1-type) domain, the region from amino acids Nos. 63 to 229 is the NMN-AT (nicotinamide mononucleotide adenylyltransferase) domain, and the region from amino acids Nos. 230 to 410 is the nicotinamide riboside kinase (ribosylnicotinamide kinase) domain.

**[Table 2]**

| Ec_NadR | Amino acid sequence | SEQ ID NO |
|---|---|---|
| Full length (410aa) | MSSFDYLKTAIKQQGCTLQQVADASGMTKGYLSQLLN AKIKSPSAQKLEALHRFLGLEFPRQKKTIGVVFGKFYPL HTGHIYLIQRACSQVDELHIIMGFDDTRDRALFEDSAM SQQPTVPDRLRWLLQTFKYQKNIRIHAFNEEGMEPYPH GWDVWSNGIKKFMAEKGIQPDLIYTSEEADAPQYMEH LGIETVLVDPKRTFMSISGAQIRENPFRYWEYIPTEVKPF FVRTVAILGGESSGKSTLVNKLANIFNTTSAWEYGRDY VFSHLGGDEIALQYSDYDKIALGHAQYIDFAVKYANK VAFIDTDFVTTQAFCKKYEGREHPFVQALIDEYRFDLVI LLENNTPWVADGLRSLGSSVDRKEFQNLLVEMLEENNI EFVRVEEEDYDSRFLRCVELVREMMGEQR* | 7 [1..410] |
| DNA binding domain | LKTAIKQQGCTLQQVADASGMTKGYLSQLLNAKIKSPS AQKLEALHRFLGLEFPRQ | 7 [7..62] |
| NMN-AT domain | KKTIGVVFGKFYPLHTGHIYLIQRACSQVDELHIIMGFD DTRDRALFEDSAMSQQPTVPDRLRWLLQTFKYQKNIRI HAFNEEGMEPYPHGWDVWSNGIKKFMAEKGIQPDLIY TSEEADAPQYMEHLGIETVLVDPKRTFMSISGAQIRENP FRYWEYIPTEVKPF | 7 [63..229] |
| NR kinase domain | FVRTVAILGGESSGKSTLVNKLANIFNTTSAWEYGRDY VFSHLGGDEIALQYSDYDKIALGHAQYIDFAVKYANK VAFIDTDFVTTQAFCKKYEGREHPFVQALIDEYRFDLVI LLENNTPWVADGLRSLGSSVDRKEFQNLLVEMLEENNI EFVRVEEEDYDSRFLRCVELVREMMGEQR | 7 [230..410] |

In the method for producing NAD according to the present embodiment, if the NMN-AT used is a DNA-binding domain-deficient mutant of Ec_NadR, the nicotinamide riboside kinase domain is (b1) or (b2) below, and the NMN-AT domain is (b3) or (b4) below. The DNA-binding domain can be (b5) or (b6) below.

(b1) a polypeptide consisting of an amino acid sequence consisting of amino acids 230 to 410 of SEQ ID NO: 7.
(b2) a polypeptide having 90% or more sequence similarity to an amino acid sequence consisting of amino acids 230 to 410 of SEQ ID NO: 7 and having nicotinamide riboside kinase activity.
(b3) a polypeptide consisting of an amino acid sequence consisting of amino acids Nos. 63 to 229 of SEQ ID NO: 7, or
(b4) a polypeptide having 90% or more sequence similarity to an amino acid sequence consisting of amino acids Nos. 63 to 229 of SEQ ID NO: 7 and having an NMN-AT activity.
(b5) a polypeptide consisting of an amino acid sequence represented by the amino acid sequence consisting of amino acids Nos. 7 to 62 of SEQ ID NO: 7.
(b6) a polypeptide having 90% or more sequence similarity to the amino acid sequence consisting of amino acids Nos. 7 to 62 of SEQ ID NO: 7 and having DNA-binding activity.

In the amino acid sequence of the polypeptide (b2) described above, the sequence similarity to the amino acid sequence consisting of amino acids Nos. 230 to 410 of SEQ ID NO: 7 is not particularly limited as long as it is 90% or more and less than 100%, and it is preferably 95% or more and less than 100%, and more preferably 98% or more and less than 100%. Furthermore, in the amino acid sequence of (b2) described above, the sequence similarity to the amino acid sequence consisting of amino acids Nos. 230 to 410 of SEQ ID NO: 7 may be 70% or more, or 80% or more.

The sequence similarity of the amino acid sequence of the polypeptide (b4) to the amino acid sequence consisting of amino acids Nos. 63 to 229 of SEQ ID NO: 7 is not particularly limited as long as it is 90% or more but less than 100%, and is preferably 95% or more but less than 100%, and more preferably 98% or more but less than 100%. Furthermore, the sequence similarity of the amino acid sequence of the polypeptide (b4) to the amino acid sequence consisting of amino acids Nos. 63 to 229 of SEQ ID NO: 7 may be 70% or more, or may be 80% or more.

The sequence similarity of the amino acid sequence of the polypeptide (b6) to the amino acid sequence consisting of amino acids 7 to 62 of SEQ ID NO: 7 is not particularly limited as long as it is 90% or more but less than 100%, and is preferably 95% or more but less than 100%, and more preferably 98% or more but less than 100%. Furthermore, the sequence similarity of the amino acid sequence of the polypeptide (b6) to the amino acid sequence consisting of amino acids Nos. 7 to 62 of SEQ ID NO: 7 may be 70% or more, or may be 80% or more.

The polypeptide (b2) may be a polypeptide having 90% to less than 100%, preferably 95% to less than 100%, and more preferably 98% to less than 100% sequence identity to the amino acid sequence consisting of amino acids Nos. 230 to 410 of SEQ ID NO: 7, and having nicotinamide riboside kinase activity.

The polypeptide (b4) may be a polypeptide having 90% to less than 100%, preferably 95% to less than 100%, and more preferably 98% to less than 100% sequence identity to the amino acid sequence consisting of amino acids Nos. 63 to 229 of SEQ ID NO:7, and having NMN-AT activity.

The polypeptide (b6) may be a polypeptide having 90% to less than 100%, preferably 95% to less than 100%, and more preferably 98% to less than 100% sequence identity to the amino acid sequence consisting of amino acids Nos. 7 to 62 of SEQ ID NO: 7, and having DNA-binding activity.

By replacing the DNA-binding domain-deficient mutant of Ec_NadR from Escherichia coli with a DNA-binding domain-deficient mutant of NadR derived from bacteria such as Salmonella typhimurium and Haemophilus ducreyi, a similar effect of improving NAD yield can be obtained.

In the method for producing NAD in the embodiment, NAD is synthesized by a transformed microorganism (hereinafter sometimes referred to as "NAD-producing microorganism") into which a gene encoding a protein having a NAMPT catalytic region consisting of one of the polypeptides (a1) to (a4) and a modified nadR gene have been introduced into the host bacterium. The NAD-producing microorganism can be produced in the same manner as the NAMPT-introduced microorganism described above, except for the introduction of the modified nadR gene.

The modified nadR gene is not particularly limited, and it is preferable to select a degenerate codon that is frequently used by the host. The preparation of an expression vector incorporating the modified nadR gene in a state that allows it to be expressed in host cells, and its introduction into the host cells , can be carried out in the same manner as the NAMPT-introduced microorganism described above.

Either the expression vector for the modified nadR gene or the expression vector for the protein having a NAMPT catalytic region consisting of any one of the polypeptides (a1) to (a4) may be introduced into the host cells first, or they may be introduced into the host simultaneously. Alternatively, an expression vector incorporating both an expression cassette containing the modified nadR gene and an expression cassette containing a gene encoding a protein having NAMPT catalytic region consisting of any one of the polypeptides (a1) to (a4) may be introduced into the host.

The microorganism used for NAD production in the present embodiment preferably incorporates, in addition to a gene encoding a protein having NAMPT catalytic region consisting of any polypeptide (a1) to (a4) and a modified nadR gene, an exogenous or endogenous transporter gene that can be expressed. When the exogenous transporter gene is expressed, or when the endogenous transporter is expressed in greater quantities than the host bacteria, the NAD synthesized within the cell of the microorganism for NAD production is secreted outside the cell. This allows for the easy acquisition of relatively high-purity NAD simply by collecting the culture medium.

The exogenous or endogenous transporter gene is not particularly limited as long as it is a transporter capable of secreting NAD from the microorganism for NAD production, and examples thereof include transporters known to use NAD as a substrate, and multidrug efflux transporters (MFS transporters) classified as MFS superfamily. MFS transporters are monomeric proteins capable of transporting small amounts of solute in response to a proton gradient. Most of these proteins consist of 400 to 600 amino acids and have 12 transmembrane α-helix regions (TM) linked by hydrophilic loops (Non-Patent Documents 5 and 6). Among the transporters used in the present embodiment, which may have exogenous or endogenous genes in the NAD-producing microorganism, MFS transporters are particularly preferable.

Specific examples of transporters other than the MFS transporter, for which the NAD-producing microorganism used in the present embodiment may have an exogenous or endogenous gene, include the region of amino acids Nos. 82 to 366 (SEQ ID NO: 8) of Yia6p/Ndtip from yeast (Todisco et al., J Biol Chem., 2006, vol. 281(3), p. 1524-1531.), which is a transporter known to use NAD as a substrate. Specific examples of the MFS transporter, for which the NAD-producing microorganism used in the present embodiment may have an exogenous or endogenous gene, include the region of amino acids Nos. 52 to 252 (SEQ ID NO: 13) of Ntt4 from Chlamydia (Haferkamp et al., Nature, 2004, vol. 432(7017), p. 622-625.), which is an MFS transporter known to use NAD as a substrate; the region of amino acids Nos. 19 to 398 (SEQ ID NO: 10) of MdfA from Escherichia coli, which is an MFS-type multidrug efflux transporter; the region of amino acids 16 to 451 (SEQ ID NO: 11) of HsrA, which is also an MFS transporter from Escherichia coli; the region of amino acids Nos. 54 to 450 (referred to as Cs_Mfs1) (SEQ ID NO: 9) of an MFS transporter from the coryneform bacterium Corynebacterium stationis; the region of amino acids 2 to 288 (Cs_Mfs2) (SEQ ID NO: 13) of another MFS transporter from Corynebacterium stationis; the region of amino acids Nos. 26 to 422 (Ctu_Mfs) (SEQ ID NO: 15) of an MFS transporter from Corynebacterium tuberculostearicum; the region of amino acids Nos. 18 to 414 (Cspp_Mfs) (SEQ ID NO: 17) of an MFS transporter from Corynebacterium sp. p3-SID1194; the region of amino acids Nos. 25 to 421 (Cge_Mfs) (SEQ ID NO: 19) of an MFS transporter from Corynebacterium genitalium; the region of amino acids Nos. 54 to 450 (Cgl_Mfs1) (SEQ ID NO: 21) of an MFS transporter from Corynebacterium glutamicum; the region of amino acids Nos. 50 to 445 (Cgl_Mfs2) (SEQ ID NO: 23) of another MFS transporter from Corynebacterium glutamicum; the region of amino acids Nos. 47 to 443 (Cpt_Mfs2) (SEQ ID NO: 25) of an MFS transporter from Corynebacterium pseudotuberculosis; the region of amino acids Nos. 68 to 463 (Ckr_Mfs) (SEQ ID NO: 27) of an MFS transporter from Corynebacterium kroppenstedtii; and the like.

Because of the high efficiency of NAD secretion outside the cell of bacterium, the MFS transporter used in the present embodiment for NAD production may have exogenous or endogenous genes, and an MFS transporter having an SLDQ motif or SLEQ motif in the first transmembrane region is particularly preferable. Among MFS transporters, MFS transporters having an SLGQ motif in the first transmembrane region have high NAD secretion efficiency. Furthermore, among MFS transporters having an SLGQ motif, MFS transporters in which this G (glycine residue) is substituted with an acidic amino acid residue D (aspartic acid residue) or E (glutamic acid residue) have even higher NAD secretion efficiency.

As exogenous or endogenous transporters expressed in the microorganisms for NAD production, NAD secretion ability is maintained even if the first S (serine), second L (leucine), and fourth Q (glutamine) in the SLDQ motif or SLEQ motif of the natural MFS transporter are replaced with amino acids that have similar function and structure. Examples of the amino acids similar to S include T (threonine), examples of amino acids similar to L include V (valine) and I (isoleucine), and examples of amino acids similar to Q include N (asparagine). In addition to MFS transporters having the SLDQ motif or SLEQ motif, as exogenous or endogenous transporters expressed in microorganisms for NAD production, MFS transporters having any of the TLDQ motif, SIDQ motif, SVDQ motif, SLDN motif, TLEQ motif, SIEQ motif, SVEQ motif, and SLEN motif may also be used.

Many MFS transporters have conserved domain structures. The domain structure data for MFS transporters are clustered in the NCBI's CDD (Conserved Domain Database: https://www.ncbi.nlm.nih.gov/cdd) under "MFS Superfamily (accession number: cl28910)". When RPS-BLAST is performed targeting the amino acid sequence information of a protein, and the E-value is 0.01 or less, if the domain model in cl28910 is hit, then that protein can be considered an MFS transporter. RPS-BLAST can be performed using known programs such as CD-Search (https://www.ncbi.nlm.nih.gov/Structure/cdd/wrpsb.cgi).

MFS transporters having an SLDQ motif or SLEQ motif in the first transmembrane domain may be natural MFS transporters such as Cgl_Mfs2, Cpt_Mfs2, and Ckr_Mfs2, or may be mutants in which the SLGQ motif in a natural MFS transporter has been replaced with an SLDQ motif or SLEQ motif. Examples of such mutants include Cs_Mfs1^{G64E} represented by SEQ ID NO: 14, Ctu_Mfs^{G36E} represented by SEQ ID NO: 16, Cspp_Mfs^{G28E}represented by SEQ ID NO: 18, Cge_Mfs^{G35E}represented by SEQ ID NO: 20, and Cgl_Mfs1^{G64E}represented by SEQ ID NO: 22. In Tables 3 to 7, such motifs are indicated by white-on-black characters.

**[Table 3]**

| | Amino acid sequence | SEQ ID NO |
|---|---|---|
| Yia6p | LSGAFAGFLSGVAVCPLDVAKTRLQAQGLQTRFENPYYRGI MGTLSTIVRDEGPRGLYKGLVPIVLGYFPTWMIYFSVYEFSK KFFHGIFPQFDFVAQSCAAITAGAASTTLTNPIWVVKTRLML QSNLGEHPTHYKGTFDAFRKLFYQEGFKALYAGLVPSLLGL FHVAIHFPIYEDLKVRFHCYSRENNTNSINLQRLIMASSVSKM | 8 |
| | IASAVTYPHEILRTRMQLKSDIPDSIQRRLFPLIKATYAQEGL KGFYSGFTTNLVRTIPASAITLVSFEYFRNRLE | |
| Ntt4 | VFFTATFLATLVLTPLFGWLVSCWPRRVIMPLIYLFFIALQM VFILLFLNKNLLSPKVLGIFFYVWVSIFNLFVVSVFWSFMTDI WSDPQARRLFPIIGLGGTLGAVMGPIITRSLVEFIGLALLLAV SAAFLAIAVICIMLLGKWAHDHGAHRNELGSESAIGGGMLD GLKQIFSNPFIASMSLMMLLNDAIGTIAYVLI | 9 |
| MdfA | PLCLVLYEFSTYIGNDMIQPGMLAVVEQYQAGIDWVPTSMT AYLAGGMFLQWLLGPLSDRIGRRPVMLAGVVWFIVTCLAIL LAQNIEQFTLLRFLQGISLCFIGAVGYAAIQESFEEAVCIKITA LMANVALIAPLLGPLVGAAWIHVLPWEGMFVLFAALAAISF FGLQRAMPETATRIGEKLSLKELGRDYKLVLKNGRFVAGAL ALGFVSLPLLAWIAQSPIIIITGEQLSSYEYGLLQVPIFGALIAG NLLLARLTSRRTVRSLIIMGGWPIMIGLLVAAAATVISSHAYL WMTAGLSIYAFGIGLANAGLVRLTLFASDMSKGTVSAAMG MLQMLIFTVGIEISKHAWLNGGNGLFNLFNLVNGILWLSLM VIF | 10 |
| HsrA | AAMAFFMQALDATILNTALPAIAHSLNRSPLAMQSAIISYTL TVAMLIPVSGWLADRFGTRRIFTLAVSLFTLGSLACALSNSLP QLVVFRVIQGIGGAMMMPVARLALLRAYPRNELLPVLNFVA MPGLVGPILGPVLGGVLVTWATWHWIFLINIPIGIAGLLYAR KHMPNFTTARRRFDITGFLLFGLSLVLFSSGIELFGEKIVASWI ALTVIVTSIGLLLLYILHARRTPNPLISLDLFKTRTFSIGIVGNI ATRLGTGCVPFLMPLMLQVGFGYQAFIAGCMMAPTALGSII AKSMVTQVLRRLGYRHTLVGITVIIGLMIAQFSLQSPAMAIW MLILPLFILGMAMSTQFTAMNTITLADLTDDNASSGNSVLAV TQQLSISLGVAVSAAVLRVYEGMEGTTTVEQFHYTFITMGII TVASAAMFMLLK | 11 |
| Cs_Mfs1 | LMLTMLMSSLGQMIFGSALPTIVGELGGVDQMSWVISAFMV TMTIAMPLAGQLGDRMGRKWVYISGISIFVIGSTLGGFANG MGMLITGRAIQGFGAGIMMISSQSIVAEVVSARERGKFMGIM GGVFGVSSVLGPVLGGWFTDGPGWRWGLWINIPLGLLAIIV CAFVLKLRVGEQGFKGFDWMGFAAIAITTSTLILLTTWGGSE YEWTSPTILSMAAVVIVGALLTVFIESRASQPLIPVQLFKNRN MVLTTLAGTVLGLAMMGVLGYMPTYLQMVHTLTPTEAGL MMIPMMVGMIGVSTGVGFIIAKTGNYKYYPIAGLAITAFAL WWMSQMTVETSLTGIGVRFLVFGVGLGFVMQVLVLIVQNS FPVSQVGTATAANNFFRQIGSALGASI | 12 |
| Cs_Mfs2 | SALGNSIAGIVRPWIVLERTDDPAAGLVAAAVAVPSVIFASL GGYLIDTVGRKPISVTSDIISGASVAGVVLVDQTLGLTIAWFI ALGIIGGVRDIPGMAARPALVGDVSQASGKTVDYIAGLNQAI LGVAFLVGPAVAAVLLAALNSSLVRIITAACSFLAALLTTFL RLAKRELTEKEKQAAAADNALNLQALKSWDRLSVPHPFGC LAFSFYAIGMMISGAGIAKVGTSRRRLVWAVAIGVEAIGFA MMAALGVSWLVVIGCGISGLAGGRLSPLQMVLIILSI | 13 |

**[Table 4]**

| | Amino acid sequence | SEQ ID NO |
|---|---|---|
| Cs_Mfs1^{G64E} | LMLTMLMSSLEQMIFGSALPTIVGELGGVDQMSWVISAF MVTMTIAMPLAGQLGDRMGRKWVYISGISIFVIGSTLGG FANGMGMLITGRAIQGFGAGIMMISSQSIVAEVVSARER GKFMGIMGGVFGVSSVLGPVLGGWFTDGPGWRWGLWI NIPLGLLAIIVCAFVLKLRVGEQGFKGFDWMGFAAIAITT STLILLTTWGGSEYEWTSPTILSMAAVVIVGALLTVFIES RASQPLIPVQLFKNRNMVLTTLAGTVLGLAMMGVLGY MPTYLQMVHTLTPTEAGLMMIPMMVGMIGVSTGVGFII AKTGNYKYYPIAGLAITAFALWWMSQMTVETSLTGIGV RFLVFGVGLGFVMQVLVLIVQNSFPVSQVGTATAANNF FRQIGSALGASI | 14 |
| Ctu_Mfs | LMLTMLMSSLGQMIFSSALPTIVGELGGVDHMSWVISAF LVTMTIAMPISGKLGDMLGRKWLYIGGIATFVVGSTLGG FANSMTLLIIARAVQGFGAGFMMISSQSIIAEVTSSRERG KFMGIMGGVFGLSSVLGPVLGGWFTDGPGWRWGMWM NIPLGILAIIVCTLVLHLRVGDADMKRFDWLGATFIAITT ASLILMTTWGGTEYEWGSSMILTLGAITIIGAIITVFVELR AKEPLIPVRLFKNRNMALTTLSGVVLGLAMFGVLGYMP TYLQMVHTLTPTKAGLMMIPMMVGLIGTSTGVGFIIART GHYKVYPIIGLAITAGALFWMSHLSVETSLKQLGCEFLV FGIGLGMVIQVLVLIVQNSFPLSQVGTATAANNFFRQIGS ALGASL | 15 |
| Ctu_Mfs^{G36E} | LMLTMLMSSLEQMIFSSALPTIVGELGGVDHMSWVISAF LVTMTIAMPISGKLGDMLGRKWLYIGGIATFVVGSTLGG FANSMTLLIIARAVQGFAGFMMISSQSIIAEVTSSRERG KFMGIMGGVFGLSSVLGPVLGGWFTDGPGWRWGMWM NIPLGILAIIVCTLVLHLRVGDADMKRFDWLGATFIAITT ASLILMTTWGGTEYEWGSSMILTLGAITIIGAIITVFVELR AKEPLIPVRLFKNRNMALTTLSGVVLGLAMFGVLGYMP TYLQMVHTLTPTKAGLMMIPMMVGLIGTSTGVGFIIART GHYKVYPIIGLAITAGALFWMSHLSVETSLKQLGCEFLV FGIGLGMVIQVLVLIVQNSFPLSQVGTATAANNFFRQIGS ALGASL | 16 |

**[Table 5]**

| | Amino acid sequence | SEQ ID NO |
|---|---|---|
| Cspp_Mfs | LLITMLMSSLGQMIFSSALPTIVGELGGVEHMSWVISAF | 17 |
| | MVTMTIALPLYGKLGDGLGRKWFYISGIAFFIIGSALGGF ANTMTLLIIGRAVQGFGAGAMMINSQAIIAEVVPARERG KYMGVMGAVFGISSVLGPVLGGWFTDGPGWRWGLWM NIPLGLLAMTVASIVLDLSKGNRDGFRFDWLGATFMAV STSTLILATTWGGLQYPWGSPRIVGLLVTSAVAAALFVV VELRATDPLVPMHLFRNRNMALTTAASVVLGISMTGVM AYLPTYLQMVHSMTPTEAGLMMIPMVGGMIITSIGIGAL ISRTGSYKIFPIVGLGVVALGCYLLSRLTVDTGLWTLGLY IFIYGFGLGMVMQVLILIVQNSFPIAVVGTATAANNFFRQ IGMSVGASV | |
| Cspp_Mfs^{G28 E} | LLITMLMSSLEQMIFSSALPTIVGELGGVEHMSWVISAFM VTMTIALPLYGKLGDGLGRKWFYISGIAFFIIGSALGGFA NTMTLLIIGRAVQGFGAGAMMINSQAIIAEVVPARERGK YMGVMGAVFGISSVLGPVLGGWFTDGPGWRWGLWMN IPLGLLAMTVASIVLDLSKGNRDGFRFDWLGATFMAVST STLILATTWGGLQYPWGSPRIVGLLVTSAVAALFVVVE LRATDPLVPMHLFRNRNMALTTAASVVLGISMTGVMA YLPTYLQMVHSMTPTEAGLMMIPMVGGMIITSIGIGALIS RTGSYKIFPIVGLGVVALGCYLLSRLTVDTGLWTLGLYIF IYGFGLGMVMQVLILIVQNSFPIAVVGTATAANNFFRQIG MSVGASV | 18 |
| Cge_Mfs | LLLTMLMSSLGQMIFATALPTIVGELGGVDHMSWVISAF LVTMTIAMPVNGKLGDILGRKWLYITGIGLFVVGSTIGG FAHTMELLIIGRAVQGLGAGGMMVNSQSIIAEVVPARER GKYMGVMGAVFGVSSVLGPVLGGWFTDGPGWRWAL WMNIPLGLLAMSVCAAVLRLRRGTAKGMHYDYLGTTL MVVATASLILTTTWGGTQYEWTSPTIIATSLIALVAAVAF VFVELRATNPLIPMDLFQNRNMVLTTLAGTVLGLAMTS GLAYLPTYLQMVHQLTPTEAGLMMLPMVIGMLVTGMT VGFIISKTGRYKIYPLVGMLITTLGLFLFSRLDVTTSLTRL GIYMFIFGFGLGLVMQVLVLIVQNSFPLKHVGTATSTNN FFRQIGSSIGASL | 19 |
| Cge-Mfs^{G35E} | LLLTMLMSSLEQMIFATALPTIVGELGGVDHMSWVISAF LVTMTIAMPVNGKLGDILGRKWLYITGIGLFVVGSTIGG FAHTMELLIIGRAVQGLGAGGMMVNSQSIIAEVVPARER GKYMGVMGAVFGVSSVLGPVLGGWFTDGPGWRWAL WMNIPLGLLAMSVCAAVLRLRRGTAKGMHYDYLGTTL MVVATASLILTTTWGGTQYEWTSPTIIATSLIALVAAVAF VFVELRATNPLIPMDLFQNRNMVLTTLAGTVLGLAMTS GLAYLPTYLQMVHQLTPTEAGLMMLPMVIGMLVTGMT VGFIISKTGRYKIYPLVGMLITTLGLFLFSRLDVTTSLTRL GIYMFIFGFGLGLVMQVLVLIVQNSFPLKHVGTATSTNN FFRQIGSSIGASL | 20 |

**[Table 6]**

| | Amino acid sequence | SEQ ID NO |
|---|---|---|
| Cgl_Mfs1 | LMLAMLLSSLGQTIFGSALPTIVGELGGVNHMTWVITAF LLGQTISLPIFGKLGDQFGRKYLFMFAIALFVVGSIIGALA QNMTTLIVARALQGIAGGGLMILSQAITADVTTARERAK YMGIMGSVFGLSSILGPLLGGWFTDGPGWRWGLWLNV PIGIIALVAIAVLLKLPARERGKVSVDWLGSIFMAIATTAF VLAVTWGGNEYEWASPMIIGLFITTLVAAIVFVFVEKRA VDPLVPMGLFSNRNFVLTAVAGIGVGLFMMGTIAYMPT YLQMVHGLNPTQAGLMLIPMMIGLIGTSTVVGNIVSKTG KYKWYPFIGMLIMVLALVLLSTLTPSASLALIGLYFFVFG FGLGCAMQILVLIVQNSFPITMVGTATGSNNFFRQIGGA VGSAL | 21 |
| Cgl_Mfs1^{G64E} | LMLAMLLSSLEQTIFGSALPTIVGELGGVNHMTWVITAF LLGQTISLPIFGKLGDQFGRKYLFMFAIALFVVGSIIGALA QNMTTLIVARALQGIAGGGLMILSQAITADVTTARERAK YMGIMGSVFGLSSILGPLLGGWFTDGPGWRWGLWLNV PIGIIALVAIAVLLKLPARERGKVSVDWLGSIFMAIATTAF VLAVTWGGNEYEWASPMIIGLFITTLVAAIVFVFVEKRA VDPLVPMGLFSNRNFVLTAVAGIGVGLFMMGTIAYMPT YLQMVHGLNPTQAGLMLIPMMIGLIGTSTVVGNIVSKTG KYKWYPFIGMLIMVLALVLLSTLTPSASLALIGLYFFVFG FGLGCAMQILVLIVQNSFPITMVGTATGSNNFFRQIGGA VGSAL | 22 |
| Cgl_Mfs2 | LMVAMMMASLDQMIFGTALPTIVGELGGVDHMMWVIT AYLLAETIMLPIYGKLGDLVGRKGLFIGALGIFLIGSVIGG LAGNMTWLIVGRAVQGIGGGGLMILSQAIIADVVPARER GRYMGVMGGVFGLSAVLGPLLGGWFTEGPGWRWAFW MNIPLGIIAIGVAIYFLDIPKKSVKFRWDYLGTFFMIVAAT SLILFTTWGGSQYEWSDPIIIGLIITTIVAAALLVVVELRA KDPLVPMSFFQNRNFTLTTIAGLILGIAMFGIIGYLPTYLQ MVHGINATEAGYMLIPMMVGMMGTSIWTGIRISNTGKY KLFPPIGMVVTFVALIFFARMEVSTTLWQIGIYLFVLGVG LGLAMQVLVLIVQNTLPTAVVGSATAVNNFFRQIGSSLG SAL | 23 |
| Cgl_Mfs2^{D60G} | LMVAMMMASLGQMIFGTALPTIVGELGGVDHMMWVIT AYLLAETIMLPIYGKLGDLVGRKGLFIGALGIFLIGSVIGG LAGNMTWLIVGRAVQGIGGGGLMILSQAIIADVVPARER GRYMGVMGGVFGLSAVLGPLLGGWFTEGPGWRWAFW MNIPLGIIAIGVAIYFLDIPKKSVKFRWDYLGTFFMIVAAT SLILFTTWGGSQYEWSDPIIIGLIITTIVAAALLVVVELRA KDPLVPMSFFQNRNFTLTTIAGLILGIAMFGIIGYLPTYLQ MVHGINATEAGYMLIPMMVGMMGTSIWTGIRISNTGKY KLFPPIGMVVTFVALIFFARMEVSTTLWQIGIYLFVLGVG LGLAMQVLVLIVQNTLPTAVVGSATAVNNFFRQIGSSLG SAL | 24 |

**[Table 7]**

| | Amino acid sequence | SEQ ID NO |
|---|---|---|
| Cpt_Mfs2 | LMVSMLLSSLDQMIFSTALPTIVGELGGVDHMMWVIT AYMVGETVMLPIYGKLGDLIGRKGLFIGALIVFLIGSVF GGMAHTMTMLIAGRAIQGIGAGGLMILSQAIIADIIPAR ERGRYMGWMGGIFGLSAVLGPLLGGWFTEGIGWRWA FWMNLPLGAIAVAIAIYFLRIPTLEKKPFTWDYLGTVF MIIATVSLILVTTWGGSQYEWSDPLIIGLIITTIFSAGLLV LVELKATDPLIPLSFFTNRNFALTTAAGLVLGITMFGVL GYLPTYMQMVSGISATEAGYMMIPMMVGMMGLSIWS GTRIAKTGKYRHFPIIGMLVTLGALALFHTLHTSIPLWQ IGLYLFTLGVGLGLSMQVLVLIVQNTLPMEVVGSATAV NNFFRQIGASLGAAL | 25 |
| Cpt_Mfs2^{D57G} | LMVSMLLSSLGQMIFSTALPTIVGELGGVDHMMWVIT AYMVGETVMLPIYGKLGDLIGRKGLFIGALIVFLIGSVF GGMAHTMTMLIAGRAIQGIGAGGLMILSQAIIADIIPAR ERGRYMGWMGGIFGLSAVLGPLLGGWFTEGIGWRWA FWMNLPLGAIAVAIAIYFLRIPTLEKKPFTWDYLGTVF MIIATVSLILVTTWGGSQYEWSDPLIIGLIITTIFSAGLLV LVELKATDPLIPLSFFTNRNFALTTAAGLVLGITMFGVL GYLPTYMQMVSGISATEAGYMMIPMMVGMMGLSIWS GTRIAKTGKYRHFPIIGMLVTLGALALFHTLHTSIPLWQ IGLYLFTLGVGLGLSMQVLVLIVQNTLPMEVVGSATAV NNFFRQIGASLGAAL | 26 |
| Ckr_Mfs2 | IMSAMLLASLDQMLFSTAFPTIVGDLGGVEHMSWVITS YLLCQTIMMPIYGKLGDQIGRKPIFVGAIAIFLIGSIIGGL SQSMAQLIIGRGIQGLGGGGLMILSQAIMADVVPPRER GKYAGWMGSVFGVSSVLGPVLGGWFTDGPGWRWAF WINLPVGLLAVAIALFALKLPKRGTGLKLDYFGIIFMAT SASCLILFTSWGGNQYSWSSPTIIGLEIGTVVAAAIFVFI ELHAKDPLIPMSLFKNRNFNLVTLGGLCVGIVMFGVMS YMPTYFQMVHNMNPTTSGFMMISMMVGLMGTSIGLG NVVTRTGKYRIYPPIGMLIVVASCVLLHRITVDTSLWY LGLCLFIMGVGLGMTMQILILVVQNSFPIQKVGVATGS NNFFRQIGSSLGSAL | 27 |
| Ckr_Mfs2^{D78G} | IMSAMLLASLGQMLFSTAFPTIVGDLGGVEHMSWVITS YLLCQTIMMPIYGKLGDQIGRKPIFVGAIAIFLIGSIIGGL SQSMAQLIIGRGIQGLGGGGLMILSQAIMADVVPPRER GKYAGWMGSVFGVSSVLGPVLGGWFTDGPGWRWAF WINLPVGLLAVAIALFALKLPKRGTGLKLDYFGIIFMAT SASCLILFTSWGGNQYSWSSPTIIGLEIGTVVAAAIFVFI ELHAKDPLIPMSLFKNRNFNLVTLGGLCVGIVMFGVMS | 28 |
| | YMPTYFQMVHNMNPTTSGFMMISMMVGLMGTSIGLG NVVTRTGKYRIYPPIGMLIVVASCVLLHRITVDTSLWY LGLCLFIMGVGLGMTMQILILVVQNSFPIQKVGVATGS NNFFRQIGSSLGSAL | |

In addition to the transporters listed in Table 3, the exogenous or endogenous transporters expressed in the microorganisms used for NAD production may also be transporters that have been mutated to the extent that their transporter activity is not impaired.

Specific examples of the exogenous or endogenous transporters to be expressed in the microorganism for NAD production include the polypeptides listed below (c1) to (c42).
(c1) a polypeptide consisting of an amino acid sequence represented by SEQ ID NO: 8.
(c2) a polypeptide having 90% or more sequence similarity to an amino acid sequence represented by SEQ ID NO: 8 and having NAD secretion ability.
(c3) a polypeptide consisting of an amino acid sequence represented by SEQ ID NO: 9.
(c4) a polypeptide having 90% or more sequence similarity to an amino acid sequence represented by SEQ ID NO: 9 and having NAD secretion ability.
(c5) a polypeptide consisting of an amino acid sequence represented by SEQ ID NO: 10.
(c6) a polypeptide having 90% or more sequence similarity to an amino acid sequence represented by SEQ ID NO: 10 and having NAD secretion ability.
(c7) a polypeptide consisting of an amino acid sequence represented by SEQ ID NO: 11.
(c8) a polypeptide having 90% or more sequence similarity to an amino acid sequence represented by SEQ ID NO: 11 and having NAD secretion ability.
(c9) a polypeptide consisting of an amino acid sequence represented by SEQ ID NO: 12.
(c10) a polypeptide having 90% or more sequence similarity to an amino acid sequence represented by SEQ ID NO: 12 and having NAD secretion ability.
(c11) a polypeptide consisting of an amino acid sequence represented by SEQ ID NO: 13.
(c12) a polypeptide having 90% or more sequence similarity to an amino acid sequence represented by SEQ ID NO: 13 and having NAD secretion ability.
(c13) a polypeptide consisting of an amino acid sequence represented by SEQ ID NO: 14.
(c14) a polypeptide having 90% or more sequence similarity to an amino acid sequence represented by SEQ ID NO: 14 and having NAD secretion ability.
(c15) a polypeptide consisting of an amino acid sequence represented by SEQ ID NO: 15.
(c16) a polypeptide having 90% or more sequence similarity to an amino acid sequence represented by SEQ ID NO: 15 and having NAD secretion ability.
(c17) a polypeptide consisting of an amino acid sequence represented by SEQ ID NO: 16.
(c18) a polypeptide having 90% or more sequence similarity to an amino acid sequence represented by SEQ ID NO: 16 and having NAD secretion ability.
(c19) a polypeptide consisting of an amino acid sequence represented by SEQ ID NO: 17.
(c20) a polypeptide having 90% or more sequence similarity to an amino acid sequence represented by SEQ ID NO: 17 and having NAD secretion ability.
(c21) a polypeptide consisting of an amino acid sequence represented by SEQ ID NO: 18.
(c22) a polypeptide having 90% or more sequence similarity to an amino acid sequence represented by SEQ ID NO: 18 and having NAD secretion ability.
(c23) a polypeptide consisting of an amino acid sequence represented by SEQ ID NO: 19.
(c24) a polypeptide having 90% or more sequence similarity to an amino acid sequence represented by SEQ ID NO: 19 and having NAD secretion ability.
(c25) a polypeptide consisting of an amino acid sequence represented by SEQ ID NO: 20.
(c26) a polypeptide having 90% or more sequence similarity to an amino acid sequence represented by SEQ ID NO: 20 and having NAD secretion ability.
(c27) a polypeptide consisting of an amino acid sequence represented by SEQ ID NO: 21.
(c28) a polypeptide having 90% or more sequence similarity to an amino acid sequence represented by SEQ ID NO: 21 and having NAD secretion ability.
(c29) a polypeptide consisting of an amino acid sequence represented by SEQ ID NO: 22.
(c30) a polypeptide having 90% or more sequence similarity to an amino acid sequence represented by SEQ ID NO: 22 and having NAD secretion ability.
(c31) a polypeptide consisting of an amino acid sequence represented by SEQ ID NO: 23.
(c32) a polypeptide having 90% or more sequence similarity to an amino acid sequence represented by SEQ ID NO: 23 and having NAD secretion ability.
(c33) a polypeptide consisting of an amino acid sequence represented by SEQ ID NO: 24.
(c34) a polypeptide having 90% or more sequence similarity to an amino acid sequence represented by SEQ ID NO: 24 and having NAD secretion ability.
(c35) a polypeptide consisting of an amino acid sequence represented by SEQ ID NO: 25.
(c36) a polypeptide having 90% or more sequence similarity to an amino acid sequence represented by SEQ ID NO: 25 and having NAD secretion ability.
(c37) a polypeptide consisting of an amino acid sequence represented by SEQ ID NO: 26.
(c38) a polypeptide having 90% or more sequence similarity to an amino acid sequence represented by SEQ ID NO: 26 and having NAD secretion ability.
(c39) a polypeptide consisting of an amino acid sequence represented by SEQ ID NO: 27.
(c40) a polypeptide having 90% or more sequence similarity to an amino acid sequence represented by SEQ ID NO: 27 and having NAD secretion ability.
(c41) a polypeptide consisting of an amino acid sequence represented by SEQ ID NO: 28. or
(c42) a polypeptide having 90% or more sequence similarity to an amino acid sequence represented by SEQ ID NO: 28 and having NAD secretion ability.

Examples of the polypeptides that have 90% or more sequence similarity to an amino acid sequence represented by any one of SEQ ID NOs: 12, 15, 17, 19, and 21 and have NAD secretion ability include polypeptides consisting of amino acid sequences in which the SLGQ motif in these amino acid sequences has been replaced with a TLGQ motif, a SIGQ motif, a SVGQ motif, a SLGN motif, a TLDQ motif, a SIDQ motif, a SVDQ motif, or a SLDN motif.

Examples of the polypeptides having 90% or more sequence similarity to an amino acid sequence represented by any one of SEQ ID NOs: 14, 16, 18, 20, and 22 and having NAD secretion ability include polypeptides consisting of amino acid sequences in which the SLEQ motif in these amino acid sequences has been replaced with a TLEQ motif, a SIEQ motif, a SVEQ motif, a SLEN motif, a SLDQ motif, a TLDQ motif, a SIDQ motif, a SVDQ motif, or a SLDN motif.

Examples of the polypeptides having 90% or more sequence similarity to an amino acid sequence represented by any one of SEQ ID NOs: 23, 25, and 27 and having NAD secretion ability include polypeptides consisting of amino acid sequences in which the SLDQ motif in these amino acid sequences has been replaced with a TLDQ motif, a SIDQ motif, a SVDQ motif, a SLDN motif, a SLEQ motif, a TLEQ motif, a SIEQ motif, a SVEQ motif, or a SLEN motif.

The polynucleotide encoding the exogenous or endogenous transporter is not particularly limited, and it is preferable to select a degenerate codon that is frequently used by the host. The preparation of an expression vector incorporating the gene encoding the exogenous or endogenous transporter in a state that allows it to be expressed in host cells, and its introduction into the host, can be carried out in the same manner as the NAMPT-introduced microorganism described above.

The NAD-producing microorganism used in the present embodiment can be cultured at 18 to 37°C in a culture medium containing nutrients necessary for growth, such as a carbon source, nitrogen source, and minerals, similar to the NAMPT-introduced microorganism described above. The culture medium can be the same as described above.

In the method for producing NAD in the embodiment, NMN is synthesized from Nam and PRPP within the cell of the NAD-producing microorganism using the introduced NAMPT (a protein having a NAMPT catalytic region consisting of any of the polypeptides (a1) to (a4)). NAD is synthesized from the obtained NMN and ATP using NMN-AT encoded by the modified nadR gene. Most of the synthesized NAD accumulates within the cells of bacteria, but if the NAD-producing microorganism has further introduced exogenous or endogenous transporters, the NAD is secreted outside the cells. Nam, PRPP, and ATP may be directly incorporated into the NAD-producing microorganism, or synthesized within the cell of the NAD-producing microorganism. For example, PRPP is synthesized from the glucose taken in externally by the NAD-producing microorganism using its metabolic pathway.

For example, NAD can be produced by culturing the NAD-producing microorganism in a solution containing Nam, glucose, and ATP under conditions that express the introduced NAMPT, the DNA-binding domain-deficient mutant of NadR, and exogenous or endogenous transporters. The solution may be a culture medium, and it is preferable to use a buffer containing glucose, Nam, and ATP, as this facilitates the purification of the produced NAD.

### [Examples]

The present invention will now be described in more detail with reference to the Examples, but the invention is not limited to the following examples.

### [Example 1]

In the synthesis of NMN, the reaction to synthesize NMN from PRPP and Nam is the rate-limiting step in many organisms. For this reason, Shoji et al. (Non-Patent Literature 1) selected highly active NAMPT that catalyzes this reaction. However, in the production of substances in microorganisms, it is more important to consider not only the enzyme reaction rate (Vmax) but also to determine how much of the produced substance is actually accumulated considering K_{M} and product inhibition or the like. In this regard, a new microbial-derived NadV was selected that can significantly accumulate NMN when expressed in Escherichia coli.

As candidates, in addition to Cp_NadV derived from Chitinophagaea pinensis, which Shoji et al.'s research showed to have high NMN synthesis activity, five types of NadV derived from microorganisms were selected from publicly available databases: NadV from Meiothermus ruber (Mr_NadV), Ci_NadV, NadV derived from Fructobacillus durionis (Fd_NadV), Sa_NadV, and NadV derived from Acidithiobacillus ferrivorans (Af_NadV).

DNA fragments with codons of the gene sequences encoding these NadVs optimized for Escherichia coli were artificially synthesized, and these were introduced into the pET-11a vector to create expression vectors. The obtained expression vector was used to transform Escherichia coli BL21 (DE3) strain, and the cells were cultured overnight with shaking in LB medium containing 0.1 mM IPTG at 25°C. The cells were collected and suspended in expression buffer A (30 g/L glucose, 44 mM potassium phosphate, 2 g/L nicotinamide) to an OD₆₀₀ value of 20, and cultured with shaking at 20°C for 4 hours.

After culturing, the cells were collected, and the culture medium was heated at 90°C for 5 minutes to extract components from the cells into the buffer. The amount of NMN in the buffer was measured using a commercially available measurement kit, "CycLex Nampt Colorimetric assay kit ver2" (manufactured by MBLCo., Ltd.). Specifically, the amount of NAD in the buffer was measured using an enzymatic method, then all the NMN in the buffer was converted to NAD, and then the amount of NAD was measured in the same manner as above to obtain the total amount of NAD and NMN in the buffer. The difference between the two values was considered to be the amount of NMN in the buffer.

Table 8 shows the measurement results of the NMN amount in each transformed microorganism. Ci_NadV and Sa_NadV were identified, which accumulate NMN at a higher concentration in Escherichia coli cells when expressed than Cp_NadV, which was reported to have high activity in the study by Shoji et al.. It was found that NMN can be industrially produced by extracting NMN from these microbial cells.

**[Table 8]**

| Transformed microorganism | NMN [µM] |
|---|---|
| Vector Control | 11 |
| Cp_NadV | 159 |
| Mr_NadV | 1 |
| Fd_NadV | 16 |
| Ci_NadV | 166 |
| Sa_NadV | 247 |
| Af_NadV | 18 |

### [Example 2]

The NadR protein, which catalyzes the reaction synthesizing NAD from NMN in Escherichia coli, has, in addition to its catalytic region, a region with a function of a transcriptional regulator (Non-Patent Document 3) and a function of a nicotinamide riboside kinase that synthesizes NMN from nicotinamide riboside (Non-Patent Document 4). In this regard, the following experiment was performed to clarify which domain of NadR is important for creating Escherichia coli that highly produces NAD.

DNA fragments were artificially synthesized that encode: the full-length NadR protein (SEQ ID NO: 7); a nicotinamide riboside kinase domain-deficient mutant (a protein consisting of the amino acid sequence of SEQ ID NO: 7 in which the region from amino acids Nos. 230 to 410 has been deleted); a DNA-binding domain-deficient mutant (a protein consisting of the amino acid sequence of SEQ ID NO: 7 in which the region from amino acids Nos. 7 to 62 has been deleted); and a double-deficient mutant of both the nicotinamide riboside kinase domain and the DNA-binding domain (a protein consisting of the amino acid sequence of SEQ ID NO: 7 in which the regions from amino acids Nos. 7 to 62 and from amino acids Nos. 230 to 410 have been deleted). These synthetic DNA fragments were introduced into the pSTV29 vector, and the resulting expression vector was introduced into the transformed Escherichia coli expressing Sa_NadV prepared in Example 1 for transformation.

The obtained strain was cultured overnight with shaking in LB medium containing 0.1 mM IPTG at 25°C. The microbial cells were collected and suspended in expression buffer B (30 g/L glucose, 44 mM potassium phosphate, 2 g/L nicotinamide, 2 g/L adenosine) to an OD₆₀₀ value of 20, and cultured with shaking at 20°C for 24 hours.

After the cultivation, the microbial cells were collected and the culture medium was heated at 90°C for 5 minutes to extract the components in the microbial cells into a buffer. The amounts of NMN and NAD in the buffer were measured using a commercially available assay kit, "CycLex Nampt Colorimetric assay kit ver2" (manufactured by MBL Co., Ltd.).

**[Table 9]**

| NadV | NadR | NMN [µM] | NAD [µM] |
|---|---|---|---|
| Sa_NadV | | 0 | 60 |
| Sa_NadV | Full-length protein | 30 | 65 |
| Sa_NadV | Nicotinamide riboside kinase domain-deficient mutant | 0 | 54 |
| Sa_NadV | DNA-binding domain-deficient mutant | 0 | 90 |
| Sa_NadV | Both nicotinamide riboside kinase domainand DNA-binding domain-deficient mutant | 0 | 59 |

Table 9 shows the measurement results of NAD and NMN amounts for each transformed microorganism. The results revealed that the intracellular accumulation amount of NAD was significantly increased by co-expressing a deficient mutant in which only the DNA-binding domain (transcription factor domain) was deleted, with Sa_NadV. It was demonstrated that NAD can be industrially produced by extracting NAD from these microbial cells.

### [Example 3]

Since the condensation reaction between NMN and ATP by NadR is a reversible reaction, it was thought that NAD production could be improved by secreting the accumulated NAD extracellularly. Furthermore, secreting NAD extracellularly would be industrially useful, as it would eliminate the extraction step required for NAD production. However, no transporter capable of exporting NAD, which has highly accumulated during cultivation, from the microbial cells has been discovered to date. Therefore, a search for such a transporter was conducted.

Specifically, the following were selected as candidates: Ntt4 from Chlamydia and Yia6p from yeast, which are nucleic acid-nucleic acid antiporters known to take up NAD into cells; MdfA and HsrA from Escherichia coli, which are MFS transporters with broad substrate specificity; and Cs_Mfs1 and Cs_Mfs2, which are MFS transporters from Corynebacterium stationis, a Corynebacterium bacterium with a proven track record as a nucleic acid producer.

DNA fragments encoding transporters consisting of the amino acid sequences listed in Table 3 were obtained by artificial synthesis or by PCR cloning from the genomes of each microorganism. These were introduced into the pMW218 vector, and the resulting expression vector was introduced into the transformed Escherichia coli expressing Sa_NadV and a DNA-binding domain-deficient mutant of NadR prepared in Example 2 for transformation. For comparison, transformed Escherichia coli were also prepared by introducing only the expression vector of the DNA-binding domain-deficient mutant of NadR.

The resulting strains were cultured overnight with shaking in LB medium containing 0.1 mM IPTG at 25°C. The microbial cells were collected and suspended in expression buffer B (30 g/L glucose, 44 mM potassium phosphate, 2 g/L nicotinamide, 2 g/L adenosine) to an OD₆₀₀ value of 20, and cultured with shaking at 20°C for 48 hours.

The culture supernatants were collected, and the NAD content in each culture supernatant was measured in the same manner as in Example 2.

**[Table 10]**

| | Introduced gene | NAD in culture supernatant [µM] |
|---|---|---|
| Condition 1 | Empty vector | 0 |
| Condition 2 | DNA-binding domain-deficient mutant of NadR | 0 |
| Condition 3 | DNA-binding domain-deficient mutant of NadR + Sa_nadV | 17 |
| Condition 4 | Condition 3 + ntt4 | 29 |
| Condition 5 | Condition 3 + YIA6 | 30 |
| Condition 6 | Condition 3 + mdfA | 25 |
| Condition 7 | Condition 3 + hsrA | 30 |
| Condition 8 | Condition 3 + Cs_Mfs1 | 31 |
| Condition 9 | Condition 3 + Cs_Mfs2 | 28 |

Table 10 shows the measurement results of the amount of NAD in the culture supernatant of each transformed microorganism. The results showed that the transformed microorganisms into which the selected transporters were introduced (conditions 4-9) showed a significant increase in the amount of NAD secreted into the culture supernatant compared to the transformed microorganisms under condition 3. This indicates that these transporters have the activity to secrete NAD extracellularly. It was demonstrated that NAD can be produced industrially with high efficiency by purifying these culture supernatants.

### [Example 4]

By introducing specific mutations into transporters, it is possible to increase the activity and productivity of useful substances. For Cs_Mfs1, which showed a significant effect in improving the extracellular secretion of NAD in Example 3, it was examined whether the NAD secretion capacity could be further enhanced by mutation.

Specifically, a mutation has been reported that enhances the activity of MFS transporters by enhancing interaction with the RxxQG motif conserved in their sequences (Kinose et al., Abstracts of the Annual Meeting of the Agricultural Chemical Society of Japan, 2023, 2B04-09, ISSN 2186-7976 2023). Based on this report, it was considered that the G64E mutation in Cs_Mfs1 may contribute to improving NAD export capacity. Therefore, the effect of this mutation was examined.

PCR was performed using the Cs_Mfs1 expression vector prepared in Example 3 as a template to obtain a DNA fragment in which the G64E mutation had been introduced into Cs_Mfs1 (a fragment containing a base sequence encoding the amino acid sequence (SEQ ID NO: 14) in which the 54th glycine in the amino acid sequence represented by SEQ ID NO: 12 had been replaced with glutamic acid). The resulting DNA fragment was cyclized using the "In Fusion Cloning kit" (manufactured by TaKaRa) and then introduced into Escherichia coli. From the resulting clones, clones with correctly inserted mutations were selected to obtain an expression vector for Cs_Mfs1 with the G64E mutation (Cs_Mfs1^{G64E}).

The obtained expression vector was introduced into transformed Escherichia coli expressing Sa_NadV and a DNA-binding domain-deficient mutant of nadR prepared in Example 2 for transformation. The resulting strain was cultured in the same manner as in Example 3, and the amount of NAD in the culture supernatant was measured in the same manner as in Example 3.

**[Table 11]**

| | Introduced gene | NAD in culture supernatant [µM] |
|---|---|---|
| Condition 8 | Condition 3+Cs_Mfs1 | 31 |
| Condition 10 | Condition 3+Cs_Mfs1^{G64E} | 69 |

Table 11 shows the results of measuring the amount of NAD in the culture supernatant of each transformed microorganism, along with the results for condition 8 of Example 3. As a result, under condition 10, in which Cs_Mfs1^{G64E} was expressed, the amount of NAD in the culture supernatant increased significantly compared to condition 8 of Example 3. This result indicates that the NAD secretion activity of Cs_Mfs1 was improved by introducing the G64E mutation. It was shown that more efficient industrial production of NAD is possible by purifying this culture supernatant.

### [Example 5]

The Cs_Mfs1^{G64E} used in Example 4 is an MFS transporter in which the SLGQ motif in the first transmembrane domain was replaced with a SLEQ motif. In this regard, the NAD secretion activity was examined for other MFS transporters in cases where the G in the SLGQ motif was substituted with E, an acidic amino acid.

Specifically, DNA fragments having nucleotide sequences encoding transporters that include the amino acid sequences represented by SEQ ID NOs: 16, 18, 20, and 22 shown in Tables 4 to 6 were each introduced into a pMW218 vector to obtain expression vectors for these transporters. Using the obtained expression vectors as templates, PCR was performed, and the resulting fragments were cyclized using the "In-Fusion Cloning Kit" (manufactured by TaKaRa). As a result, an expression vector encoding a transporter comprising the amino acid sequence of SEQ ID NO: 15, derived from the expression vector for a transporter comprising the amino acid sequence of SEQ ID NO: 16; an expression vector encoding a transporter comprising the amino acid sequence of SEQ ID NO: 17, derived from the expression vector for a transporter comprising the amino acid sequence of SEQ ID NO: 18; an expression vector encoding a transporter comprising the amino acid sequence of SEQ ID NO: 19, derived from the expression vector for a transporter comprising the amino acid sequence of SEQ ID NO: 20; and an expression vector encoding a transporter comprising the amino acid sequence of SEQ ID NO: 21, derived from the expression vector for a transporter comprising the amino acid sequence of SEQ ID NO: 22, were obtained.

The obtained expression vectors were introduced into transformed Escherichia coli expressing Sa_NadV and a DNA-binding domain-deficient mutant of nadR prepared in Example 2 for transformation. The obtained strains were cultured in the same manner as in Example 3, and the amounts of NAD in the culture supernatants were measured in the same manner as in Example 3.

**[Table 12]**

| | Introduced gene | NAD in culture supernatant (Relative value) |
|---|---|---|
| Condition 3 | DNA-binding domain-deficient mutant of NadR + Sa_nadV | 1.0 ± 0.02 |
| Condition 8 | Condition 3 + Cs_Mfs1 | 2.1 ± 0.3 |
| Condition 10 | Condition 3 + Cs_Mfs1 G64E | 9.0 ± 0.5 |
| Condition 11 | Condition 3 + Ctu_Mfs | 1.9 ± 0.2 |
| Condition 12 | Condition 3 + Ctu_Mfs ^{G36E} | 5.8 ± 0.5 |
| Condition 13 | Condition 3 + Cspp_Mfs | 8.2 ± 0.5 |
| Condition 14 | Condition 3 + Cspp_Mfs^{G28E} | 15.6 ± 2.1 |
| Condition 15 | Condition 3 + Cge_Mfs | 8.1 ± 1.7 |
| Condition 16 | Condition 3 + Cge_Mfs^{G35E} | 17.7 ± 1.4 |
| Condition 17 | Condition 3 + Cgl_Mfs1 | 2.8 ± 0.1 |
| Condition 18 | Condition 3 + Cgl_Mfs1^{G64E} | 5.9 ± 1.7 |

The amounts of NAD in the culture supernatants under each condition are shown in Table 12 as relative values, with the amount of NAD in the culture supernatant under the control condition 3 set to 1.0. The values in Table 12 are the average value ± standard deviation of three independent trials. In all of the MFS transporters, the amount of NAD in the culture supernatant was higher when the MFS transporter having the SLEQ motif, in which G was substituted with an acidic amino acid E, was expressed, compared to when the corresponding MFS transporter having the wild-type SLGQ motif was expressed.

### [Example 6]

The NAD secretion activity was examined for a wild-type MFS transporter containing the SLDQ motif in its first transmembrane region. Furthermore, the effect on NAD secretion activity was also examined in the case where the acidic amino acid D in the motif was substituted with G, a neutral amino acid.

DNA fragments having nucleotide sequences encoding transporters that contain the amino acid sequences represented by SEQ ID NOs: 23, 25, and 27 shown in Tables 6 to 7 were each introduced into a pMW218 vector to obtain expression vectors for these transporters. Using the obtained expression vectors as templates, PCR was performed, and the resulting fragments were cyclized using the "In-Fusion Cloning Kit" (manufactured by TaKaRa). As a result, an expression vector encoding a transporter containing the amino acid sequence of SEQ ID NO: 24 from the expression vector for the transporter comprising the amino acid sequence of SEQ ID NO: 23, an expression vector encoding a transporter comprising the amino acid sequence of SEQ ID NO: 26 from the expression vector for the transporter comprising the amino acid sequence of SEQ ID NO: 25, and an expression vector encoding a transporter comprising the amino acid sequence of SEQ ID NO: 28 from the expression vector for the transporter comprising the amino acid sequence of SEQ ID NO: 27 were obtained.

The obtained expression vectors were introduced into transformed Escherichia coli expressing Sa_NadV and a DNA-binding domain-deficient mutant of nadR prepared in Example 2. The obtained strains were cultured in the same manner as in Example 3, and the amounts of NAD in the culture supernatants were measured in the same manner as in Example 3.

**[Table 13]**

| Introduced gene | | NAD in culture supernatant (Relative value) |
|---|---|---|
| Condition 3 | DNA-binding domain-deficient mutant of NadR + Sa_nadV | 1.0 ± 0.4 |
| Condition 19 | Condition 3 + Cgl_Mfs2 | 3.3 ± 0.3 |
| Condition 20 | Condition 3 + Cgl_Mfs2^{D60G} | 1.1 ± 0.3 |
| Condition 21 | Condition 3 + Cpt_Mfs2 | 11.2 ± 1.1 |
| Condition 22 | Condition 3 + Cpt_Mfs2^{D57G} | 1.3 ± 0.5 |
| Condition 23 | Condition 3 + Ckr_Mfs1 | 4.0 ± 0.5 |
| Condition 24 | Condition 3 + Ckr_Mfs1^{D78G} | 1.0 ± 0.1 |

The amounts of NAD in the culture supernatants under each condition are shown in Table 13 as a relative values, with the amount of NAD in the culture supernatant under the control condition 3 set to 1.0. The values in Table 13 are the average value ± standard deviation of three independent trials. In all of the MFS transporters, the amount of NAD in the culture supernatant was higher when the MFS transporter having the wild-type SLDQ motif was expressed, compared to when the corresponding MFS transporter having the SLGQ motif, in which D was substituted with G, was expressed.

## Claims

1. A method for producing nicotinamide mononucleotide, which involves synthesizing the nicotinamide mononucleotide within bacterial cells, comprising:
synthesizing nicotinamide mononucleotide from nicotinamide and phosphoribosyl pyrophosphate using nicotinamide phosphoribosyltransferase, wherein
the nicotinamide phosphoribosyltransferase comprises a region consisting of:
(a1) a polypeptide having an amino acid sequence represented by SEQ ID NO: 2 and having, on C-terminal of said amino acid sequence, an amino acid sequence represented by SEQ ID NO: 3,
(a2) a polypeptide having an amino acid sequence having 90% or more sequence similarity to an amino acid sequence represented by SEQ ID NO: 2 and having, on C-terminal of said amino acid sequence, an amino acid sequence having 90% or more sequence similarity to an amino acid sequence represented by SEQ ID NO: 3, and having nicotinamide phosphoribosyltransferase activity,
(a3) a polypeptide having an amino acid sequence represented by SEQ ID NO: 5 and having, on C-terminal of said amino acid sequence, an amino acid sequence represented by SEQ ID NO: 6, or
(a4) a polypeptide having an amino acid sequence having 90% or more sequence similarity to an amino acid sequence represented by SEQ ID NO: 5 and having, on C-terminal of said amino acid sequence, an amino acid sequence having 90% or more sequence similarity to an amino acid sequence represented by SEQ ID NO: 6, and having nicotinamide phosphoribosyltransferase activity.

2. The method for producing nicotinamide mononucleotide according to claim 1, wherein the bacteria are cultured in a solution containing glucose and nicotinamide.

3. A method for producing nicotinamide adenine dinucleotide, which involves synthesizing the nicotinamide adenine dinucleotide within bacterial cells, comprising:
synthesizing nicotinamide mononucleotide from nicotinamide and phosphoribosyl pyrophosphate using nicotinamide phosphoribosyltransferase, and
synthesizing nicotinamide adenine dinucleotide from the obtained nicotinamide mononucleotide and ATP using nicotinamide mononucleotide adenylyltransferase,
wherein
the nicotinamide phosphoribosyltransferase comprises a region consisting of:
(a1) a polypeptide having an amino acid sequence represented by SEQ ID NO: 2 and having, on C-terminal of said amino acid sequence, an amino acid sequence represented by SEQ ID NO: 3,
(a2) a polypeptide having an amino acid sequence having 90% or more sequence similarity to an amino acid sequence represented by SEQ ID NO: 2 and having, on C-terminal of said amino acid sequence, an amino acid sequence having 90% or more sequence similarity to an amino acid sequence represented by SEQ ID NO: 3, and having nicotinamide phosphoribosyltransferase activity,
(a3) a polypeptide having an amino acid sequence represented by SEQ ID NO: 5 and having, on C-terminal of said amino acid sequence, an amino acid sequence represented by SEQ ID NO: 6, or
(a4) a polypeptide having an amino acid sequence having 90% or more sequence similarity to an amino acid sequence represented by SEQ ID NO: 5 and having, on C-terminal of said amino acid sequence, an amino acid sequence having 90% or more sequence similarity to an amino acid sequence represented by SEQ ID NO: 6, and having nicotinamide phosphoribosyltransferase activity;
the nicotinamide mononucleotide adenylyltransferase is encoded in a modified nadR gene introduced into the bacterium, and the modified nadR gene encodes a polypeptide that has a nicotinamide riboside kinase domain and a nicotinamide mononucleotide adenylyltransferase domain, but does not have a DNA-binding domain;
the nicotinamide riboside kinase domain is:
(b1) a polypeptide consisting of an amino acid sequence consisting of amino acids Nos. 230 to 410 of SEQ ID NO: 7, or
(b2) a polypeptide having 90% or more sequence similarity to an amino acid sequence consisting of amino acids Nos. 230 to 410 of SEQ ID NO: 7 and having nicotinamide riboside kinase activity;
the nicotinamide mononucleotide adenylyltransferase domain is:
(b3) a polypeptide consisting of an amino acid sequence consisting of amino acids Nos. 63 to 229 of SEQ ID NO: 7, or
(b4) a polypeptide having 90% or more sequence similarity to an amino acid sequence consisting of amino acids Nos. 63 to 229 of SEQ ID NO: 7 and having nicotinamide mononucleotide adenylyltransferase activity.

4. The method for producing nicotinamide adenine dinucleotide according to claim 3, wherein the bacteria express an exogenous or endogenous transporter gene, and the synthesized nicotinamide adenine dinucleotide is secreted outside the bacterium.

5. The method for producing nicotinamide adenine dinucleotide according to claim 4, wherein
the transporter is:
(c1) a polypeptide consisting of an amino acid sequence represented by SEQ ID NO: 8,
(c2) a polypeptide having 90% or more sequence similarity to an amino acid sequence represented by SEQ ID NO: 8 and having nicotinamide adenine dinucleotide secretion ability,
(c3) a polypeptide consisting of an amino acid sequence represented by SEQ ID NO: 9,
(c4) a polypeptide having 90% or more sequence similarity to an amino acid sequence represented by SEQ ID NO: 9 and having nicotinamide adenine dinucleotide secretion ability,
(c5) a polypeptide consisting of an amino acid sequence represented by SEQ ID NO: 10,
(c6) a polypeptide having 90% or more sequence similarity to an amino acid sequence represented by SEQ ID NO: 10 and having nicotinamide adenine dinucleotide secretion ability,
(c7) a polypeptide consisting of an amino acid sequence represented by SEQ ID NO: 11,
(c8) a polypeptide having 90% or more sequence similarity to an amino acid sequence represented by SEQ ID NO: 11 and having nicotinamide adenine dinucleotide secretion ability,
(c9) a polypeptide consisting of an amino acid sequence represented by SEQ ID NO: 12,
(c10) a polypeptide having 90% or more sequence similarity to an amino acid sequence represented by SEQ ID NO: 12 and having nicotinamide adenine dinucleotide secretion ability,
(c11) a polypeptide consisting of an amino acid sequence represented by SEQ ID NO: 13,
(c12) a polypeptide having 90% or more sequence similarity to an amino acid sequence represented by SEQ ID NO: 13 and having nicotinamide adenine dinucleotide secretion ability,
(c13) a polypeptide consisting of an amino acid sequence represented by SEQ ID NO: 14,
(c14) a polypeptide having 90% or more sequence similarity to an amino acid sequence represented by SEQ ID NO: 14 and having nicotinamide adenine dinucleotide secretion ability,
(c15) a polypeptide consisting of an amino acid sequence represented by SEQ ID NO: 15,
(c16) a polypeptide having 90% or more sequence similarity to an amino acid sequence represented by SEQ ID NO: 15 and having nicotinamide adenine dinucleotide secretion ability,
(c17) a polypeptide consisting of an amino acid sequence represented by SEQ ID NO: 16,
(c18) a polypeptide having 90% or more sequence similarity to an amino acid sequence represented by SEQ ID NO: 16 and having nicotinamide adenine dinucleotide secretion ability,
(c19) a polypeptide consisting of an amino acid sequence represented by SEQ ID NO: 17,
(c20) a polypeptide having 90% or more sequence similarity to an amino acid sequence represented by SEQ ID NO: 17 and having nicotinamide adenine dinucleotide secretion ability,
(c21) a polypeptide consisting of an amino acid sequence represented by SEQ ID NO: 18,
(c22) a polypeptide having 90% or more sequence similarity to an amino acid sequence represented by SEQ ID NO: 18 and having nicotinamide adenine dinucleotide secretion ability,
(c23) a polypeptide consisting of an amino acid sequence represented by SEQ ID NO: 19,
(c24) a polypeptide having 90% or more sequence similarity to an amino acid sequence represented by SEQ ID NO: 19 and having nicotinamide adenine dinucleotide secretion ability,
(c25) a polypeptide consisting of an amino acid sequence represented by SEQ ID NO: 20,
(c26) a polypeptide having 90% or more sequence similarity to an amino acid sequence represented by SEQ ID NO: 20 and having nicotinamide adenine dinucleotide secretion ability,
(c27) a polypeptide consisting of an amino acid sequence represented by SEQ ID NO: 21,
(c28) a polypeptide having 90% or more sequence similarity to an amino acid sequence represented by SEQ ID NO: 21 and having nicotinamide adenine dinucleotide secretion ability,
(c29) a polypeptide consisting of an amino acid sequence represented by SEQ ID NO: 22,
(c30) a polypeptide having 90% or more sequence similarity to an amino acid sequence represented by SEQ ID NO: 22 and having nicotinamide adenine dinucleotide secretion ability,
(c31) a polypeptide consisting of an amino acid sequence represented by SEQ ID NO: 23,
(c32) a polypeptide having 90% or more sequence similarity to an amino acid sequence represented by SEQ ID NO: 23 and having nicotinamide adenine dinucleotide secretion ability,
(c33) a polypeptide consisting of an amino acid sequence represented by SEQ ID NO: 24,
(c34) a polypeptide having 90% or more sequence similarity to an amino acid sequence represented by SEQ ID NO: 24 and having nicotinamide adenine dinucleotide secretion ability,
(c35) a polypeptide consisting of an amino acid sequence represented by SEQ ID NO: 25,
(c36) a polypeptide having 90% or more sequence similarity to an amino acid sequence represented by SEQ ID NO: 25 and having nicotinamide adenine dinucleotide secretion ability,
(c37) a polypeptide consisting of an amino acid sequence represented by SEQ ID NO: 26,
(c38) a polypeptide having 90% or more sequence similarity to an amino acid sequence represented by SEQ ID NO: 26 and having nicotinamide adenine dinucleotide secretion ability,
(c39) a polypeptide consisting of an amino acid sequence represented by SEQ ID NO: 27,
(c40) a polypeptide having 90% or more sequence similarity to an amino acid sequence represented by SEQ ID NO: 27 and having nicotinamide adenine dinucleotide secretion ability,
(c41) a polypeptide consisting of an amino acid sequence represented by SEQ ID NO: 28, or
(c42) a polypeptide having 90% or more sequence similarity to an amino acid sequence represented by SEQ ID NO: 28 and having nicotinamide adenine dinucleotide secretion ability.

6. The method for producing nicotinamide adenine dinucleotide according to claim 4, wherein the transporter is MFS transporter having SLDQ motif or SLEQ motif in the first transmembrane domain.

7. A transformed microorganism, which is a microbial transformant, into which an exogenous nicotinamide phosphoribosyltransferase gene has been introduced, wherein
the nicotinamide phosphoribosyltransferase comprises a region consisting of:
(a1) a polypeptide having an amino acid sequence represented by SEQ ID NO: 2 and having, on C-terminal of said amino acid sequence, an amino acid sequence represented by SEQ **ID** NO: 3,
(a2) a polypeptide having an amino acid sequence having 90% or more sequence similarity to an amino acid sequence represented by SEQ ID NO: 2 and having, on C-terminal of said amino acid sequence, an amino acid sequence having 90% or more sequence similarity to an amino acid sequence represented by SEQ ID NO: 3, and having nicotinamide phosphoribosyltransferase activity,
(a3) a polypeptide having an amino acid sequence represented by SEQ ID NO: 5 and having, on C-terminal of said amino acid sequence, an amino acid sequence represented by SEQ **ID** NO: 6, or
(a4) a polypeptide having an amino acid sequence having 90% or more sequence similarity to an amino acid sequence represented by SEQ ID NO: 5 and having, on C-terminal of said amino acid sequence, an amino acid sequence having 90% or more sequence similarity to an amino acid sequence represented by SEQ ID NO: 6, and having nicotinamide phosphoribosyltransferase activity.

8. The transformed microorganism according to claim 7, wherein
a modified nadR gene has been further introduced;
the modified nadR gene encodes a polypeptide that has a nicotinamide riboside kinase domain and a nicotinamide mononucleotide adenylyltransferase domain, but does not have a DNA-binding domain;
the nicotinamide riboside kinase domain is:
(b1) a polypeptide consisting of an amino acid sequence consisting of amino acids 230 to 410 of SEQ ID NO: 7, or
(b2) a polypeptide having 90% or more sequence similarity to an amino acid sequence consisting of amino acids 230 to 410 of SEQ ID NO: 7 and having nicotinamide riboside kinase activity;
the nicotinamide mononucleotide adenylyltransferase domain is:
(b3) a polypeptide consisting of an amino acid sequence consisting of amino acids Nos. 63 to 229 of SEQ ID NO: 7, or
(b4) a polypeptide having 90% or more sequence similarity to an amino acid sequence consisting of amino acids Nos. 63 to 229 of SEQ ID NO: 7 and having nicotinamide mononucleotide adenylyltransferase activity.

9. The transformed microorganism according to claim 7 or 8, wherein
an exogenous or endogenous transporter gene has been further introduced; and
the transporter is:
(c1) a polypeptide consisting of an amino acid sequence represented by SEQ **ID** NO: 8,
(c2) a polypeptide having 90% or more sequence similarity to an amino acid sequence represented by SEQ ID NO: 8 and having nicotinamide adenine dinucleotide secretion ability,
(c3) a polypeptide consisting of an amino acid sequence represented by SEQ ID NO: 9,
(c4) a polypeptide having 90% or more sequence similarity to an amino acid sequence represented by SEQ ID NO: 9 and having nicotinamide adenine dinucleotide secretion ability,
(c5) a polypeptide consisting of an amino acid sequence represented by SEQ ID NO: 10,
(c6) a polypeptide having 90% or more sequence similarity to an amino acid sequence represented by SEQ ID NO: 10 and having nicotinamide adenine dinucleotide secretion ability,
(c7) a polypeptide consisting of an amino acid sequence represented by SEQ ID NO: 11,
(c8) a polypeptide having 90% or more sequence similarity to an amino acid sequence represented by SEQ ID NO: 11 and having nicotinamide adenine dinucleotide secretion ability,
(c9) a polypeptide consisting of an amino acid sequence represented by SEQ ID NO: 12,
(c10) a polypeptide having 90% or more sequence similarity to an amino acid sequence represented by SEQ ID NO: 12 and having nicotinamide adenine dinucleotide secretion ability,
(c11) a polypeptide consisting of an amino acid sequence represented by SEQ ID NO: 13,
(c12) a polypeptide having 90% or more sequence similarity to an amino acid sequence represented by SEQ ID NO: 13 and having nicotinamide adenine dinucleotide secretion ability,
(c13) a polypeptide consisting of an amino acid sequence represented by SEQ ID NO: 14,
(c14) a polypeptide having 90% or more sequence similarity to an amino acid sequence represented by SEQ ID NO: 14 and having nicotinamide adenine dinucleotide secretion ability,
(c15) a polypeptide consisting of an amino acid sequence represented by SEQ ID NO: 15,
(c16) a polypeptide having 90% or more sequence similarity to an amino acid sequence represented by SEQ ID NO: 15 and having nicotinamide adenine dinucleotide secretion ability,
(c17) a polypeptide consisting of an amino acid sequence represented by SEQ ID NO: 16,
(c18) a polypeptide having 90% or more sequence similarity to an amino acid sequence represented by SEQ ID NO: 16 and having nicotinamide adenine dinucleotide secretion ability,
(c19) a polypeptide consisting of an amino acid sequence represented by SEQ ID NO: 17,
(c20) a polypeptide having 90% or more sequence similarity to an amino acid sequence represented by SEQ ID NO: 17 and having nicotinamide adenine dinucleotide secretion ability,
(c21) a polypeptide consisting of an amino acid sequence represented by SEQ ID NO: 18,
(c22) a polypeptide having 90% or more sequence similarity to an amino acid sequence represented by SEQ ID NO: 18 and having nicotinamide adenine dinucleotide secretion ability,
(c23) a polypeptide consisting of an amino acid sequence represented by SEQ ID NO: 19,
(c24) a polypeptide having 90% or more sequence similarity to an amino acid sequence represented by SEQ ID NO: 19 and having nicotinamide adenine dinucleotide secretion ability,
(c25) a polypeptide consisting of an amino acid sequence represented by SEQ ID NO: 20,
(c26) a polypeptide having 90% or more sequence similarity to an amino acid sequence represented by SEQ ID NO: 20 and having nicotinamide adenine dinucleotide secretion ability,
(c27) a polypeptide consisting of an amino acid sequence represented by SEQ ID NO: 21,
(c28) a polypeptide having 90% or more sequence similarity to an amino acid sequence represented by SEQ ID NO: 21 and having nicotinamide adenine dinucleotide secretion ability,
(c29) a polypeptide consisting of an amino acid sequence represented by SEQ ID NO: 22,
(c30) a polypeptide having 90% or more sequence similarity to an amino acid sequence represented by SEQ ID NO: 22 and having nicotinamide adenine dinucleotide secretion ability,
(c31) a polypeptide consisting of an amino acid sequence represented by SEQ ID NO: 23,
(c32) a polypeptide having 90% or more sequence similarity to an amino acid sequence represented by SEQ ID NO: 23 and having nicotinamide adenine dinucleotide secretion ability,
(c33) a polypeptide consisting of an amino acid sequence represented by SEQ ID NO: 24,
(c34) a polypeptide having 90% or more sequence similarity to an amino acid sequence represented by SEQ ID NO: 24 and having nicotinamide adenine dinucleotide secretion ability,
(c35) a polypeptide consisting of an amino acid sequence represented by SEQ ID NO: 25,
(c36) a polypeptide having 90% or more sequence similarity to an amino acid sequence represented by SEQ ID NO: 25 and having nicotinamide adenine dinucleotide secretion ability,
(c37) a polypeptide consisting of an amino acid sequence represented by SEQ ID NO: 26,
(c38) a polypeptide having 90% or more sequence similarity to an amino acid sequence represented by SEQ ID NO: 26 and having nicotinamide adenine dinucleotide secretion ability,
(c39) a polypeptide consisting of an amino acid sequence represented by SEQ ID NO: 27,
(c40) a polypeptide having 90% or more sequence similarity to an amino acid sequence represented by SEQ ID NO: 27 and having nicotinamide adenine dinucleotide secretion ability,
(c41) a polypeptide consisting of an amino acid sequence represented by SEQ ID NO: 28, or
(c42) a polypeptide having 90% or more sequence similarity to an amino acid sequence represented by SEQ ID NO: 28 and having nicotinamide adenine dinucleotide secretion ability.

10. The transformed microorganism according to claim 7 or 8, wherein
an exogenous or endogenous transporter gene has been further introduced, and
the transporter is MFS transporter having SLDQ motif or SLEQ motif in the first transmembrane domain.
